# EUROPÄISCHE PATENTSCHRIFT

(11) **EP 1 216 246 B1**
(45) Veröffentlichungstag und Bekanntmachung des Hinweises auf die Patenterteilung: **24.08.2005**
(21) Anmeldenummer: 00964154.9
(22) Anmeldetag: 11.09.2000
(51) Int. Cl.: C07D 475/08, A61K 31/519, A61P 9/00, C07D 239/50

(54) **N-SUBSTITUIERTE 4-AMINOPTERIDINE, VERFAHREN ZU IHRER HERSTELLUNG UND IHRE VERWENDUNG ALS ARZNEIMITTEL**
N-SUBSTITUTED 4-AMINOPTERIDINES, SYNTHESIS AND USE THEREOF AS PHARMACEUTICAL AGENT
4-AMINOPTERIDINE N-SUBSTITUE, SON PROCEDE DE FABRICATION ET SON UTILISATION COMME MEDICAMENT

(30) Priorität: 17.09.1999 DE 19944767
(43) Veröffentlichungstag der Anmeldung: 26.06.2002
(73) Patentinhaber: Vasopharm Biotech GmbH & Co. KG, 97082 Würzburg (DE)
(72) Erfinder: PFLEIDERER, Wolfgang, 78464 Konstanz (DE); SCHMIDT, Harald, 35392 Giessen (DE); FRÖHLICH, Lothar, 97072 Würzburg (DE); KOTSONIS, Peter, 4054 Basel (CH); TAGHAVI-MOGHADAM, Shahriyar, 78462 Konstanz (DE)
(74) Vertreter: Viering, Jentschura & Partner
(86) Internationale Anmeldenummer: PCT/EP2000/008833
(87) Internationale Veröffentlichungsnummer: WO 2001/021619

(56) Entgegenhaltungen:
- EP-A- 0 906 913
- WO-A-00/39129
- WO-A-94/14780
- DE-A- 4 120 247
- DE-A- 4 418 096
- DE-A- 4 418 097

## Beschreibung

Die vorliegende Erfindung betrifft N-substituierte 4-Aminopteridine gemäß der in den Ansprüchen definierten Formeln (Ia), (Ib) und (Ic) Verfahren zu ihrer Herstellung und die Verwendung gemäß der in den Ansprüchen definierten Formel (I) zur Vorbeugung und Behandlung von Krankheiten deren Ursache ein gestörter Stickstoffmonoxid-Haushalt ist.

Stickstoffmonoxid (NO) ist ein ubiquitärer Träger physiologischer und pathophysiologischer Funktionen (S. Moncada et al. *Pharmacol. Rev.* **43** (1991), 109-142). Es wirkt relaxierend auf die glatte Gefäßmuskulatur und ist auf diese Weise maßgeblich an der Regulation des Blutdrucks und der Proliferation von Gefäßwandzellen beteiligt; es steuert über eine Hemmung der Thrombocytenaggregation die Blutgerinnung und ist im Gehirn und Rückenmark als Neuromodulator involviert. In den NANC-Nerven des peripheren Nervensystems fungiert NO ebenfalls als Botenstoff. Die zelltoxische Wirkung des NO wird von Macrophagen und einer Vielzahl anderer Zellen für die Infektionsabwehr genutzt, spielt aber auch eine Rolle in der Entzündungsreaktion und Autoimmunreaktion.

Endogenes NO wird mit Hilfe dreier verschiedener NO-Synthase-Isoenzyme aus Arginin gebildet (Kershaw, Ann. Rep. Med. Chem. **27** (1992) 69). Alle Isoenzyme benötigen NADPH, Flavinadenindinucleotid, Flavinmononucleotid und Tetrahydrobiopterin als Cofaktoren. Sie unterscheiden sich bezüglich ihrer Lokalisation im Organismus, ihrer Regulierbarkeit durch Ca²⁺/Calmodulin sowie ihrer Induzierbarkeit durch Endotoxine und Cytokine. Die konstitutiven, calciumabhängigen NO-Synthasen finden sich beispielsweise im Endothel (Typ III) und im Gehirn (Typ I) und sind dort in die Regulation von Blutdruck- und Gerinnung bzw. in Reizleitungsprozesse involviert. Die zytokin - induzierbare, calciumunabhängige Isoform (Typ II) tritt in Macrophagen, Glattmuskelzellen und Hepatocyten auf Sie ist in der Lage, über einen langen Zeitraum relativ große Mengen NO zu produzieren und wird für entzündliche und Autoimmun-Prozesse und die zelltoxische Aktivität der Macrophagen verantwortlich gemacht.

Ein gestörter NO-Haushalt hat schwerwiegende Erkrankungen und Schädigungen zur Folge. So führt die exzessive Bildung von NO im septischen oder hämorrhagischen Schock zu massiven pathologischen Blutdruckabfällen. Übersteigerte NO-Produktion ist z.B. an der Entstehung von Autoimmunerkrankungen, wie Typ-1-Diabetes sowie von Atherosklerose beteiligt und mitverantwortlich für die Glutamat-induzierte Neurotoxizität nach cerebraler Ischämie. Hohe NO-Konzentrationen können darüber hinaus durch Desaminierung von Cytosin zu DNA-Schädigungen und Krebs führen. Eine selektive Hemmung der an den jeweiligen Krankheitsbildern beteiligten NO-Synthasen eignet sich deshalb zur Behandlung und Vorbeugung der genannten Krankheiten.

Nur wenige Vertreter N-substituierter 4-Aminopterine sind bisher in der chemischen Literatur bekannt. Alle diese Vertreter enthalten entweder einen von Wasserstoff verschiedenen Substituenten in der 7-Postion des Pterin-Gerüsts oder einen der Folsäure analogen Aminobenzoylglutamat-Rest in der 6-Position des Pterin-Gerüsts (siehe nachfolgende Formeln (a) und (b) bezüglich Pterin-Gerüst).

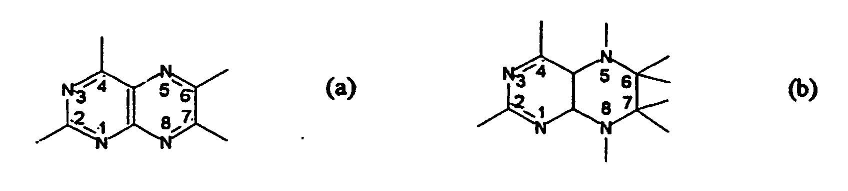

Über die pharmakologische Wirkung N-substituierter 4-Aminopterine existiert nur äußerst wenig Information: Dewey et al. (Biochem. Parmacol. **23** (1974) 773) und Weinstock et aL (J. Med. Chem. **11** (1968) 573) berichten von einer potentiell diuretischen Wirkung von 2,7-Diamino-4-methylamino-6-phenylpteridin, Roth et al. (J. Am. Chem. Soc. **73** (1951) 1914) bestimmten die antagonistische Wirkung verschiedener folsäureanaloger (2-Amino-4-alkylamino-pteridin-6-ylmethyl)-aminobenzoylglutamate auf S.faecalis R. Die Wirkung dieser Derivate, die von den Autoren als "schwach" charakterisiert wird, dürfte in hohem Maße an das Vorhandensein des für solche Wirkstoffe typischen Aminobenzoylglutamtats in der 6-Position des Pteridins zurückzuführen sein.

Die Internationale Patentanmeldung WO 00/39129, die als Europäische Patentanmeldung 99 964 663 weitergeführt wurde und ein Dokument darstellt, das nach Art. 54(3) EPÜ zu berücksichtigen ist, offenbart N-substituierte 4-Aminopteridine, die gemäß dieser Patentanmeldung zur Behandlung von Autoimmunerkrankungen oder Transplantatabstoßungen verwendet werden können.

Die Verwendung von Pterin-Analoga zur Hemmung der NO-Synthase (NOS) wurde in der Literatur bislang ebenfalls nur wenig diskutiert. Die Mehrzahl aller publizierten pharmakologischen Ansätze zur NOS-Hemmung basieren auf einer kompetitiven Beeinflussung der Substrat-Bindungsstelle des Enzyms für L-Arginin über Substratanaloga (siehe etwa E.S. Furfine et al. J. Biol. Chem. 269 (1994) 26677).

Als weitere potentielle NO-Synthase-Hemmer wurden in der Literatur N-Iminoethylornithin (Mc Call et al., Br. J. Pharmacol. 102 (1991) 234), Aminoguanidin (T.P. Misko et al., Eur. J.-Pharmacol., 233 (1993) 119, EP547588-A1) und 7-Nitroindazol (P.K. Moore et al., Br. J. Pharmacol. 108 (1993) 296) diskutiert.

Die Wirkung einfacher 6R-5,6,7,8-Tetrahydrobiopterin-Analoga (BH₄-Analoga) auf die NO-Produktion wurde von Stuehr et al. (J. Biol. Chem. 264 (1989) 20496), Giovanelli et al. (Proc. Natl. Acad. Sci. **88** (1991) 7091), Mülsch und Busse (J. Cardiovasc. Pharmacol. 17 (1991) S52), Sakai et al. (Mol. Pharmacol. **43** (1992) 6), Werner et al.(FEBS Letters 305 (1992) 160), Wachter et al. (Biochem. J. 289 (1993) 357) sowie von Hevel und Marletta (Biochemistry **31** (1992) 7160) untersucht. Danach sind 6S-BH₄, 7-R/S-BH₄, 6-Methyl-5,6,7,8-tetrahydropterin und Dihydrobiopterin in der Lage, den natürlichen Cofaktor teilweise zu ersetzen. Biopterin, 6,7-Dimethyl-5,6,7,8-tetrahydropterin, Tetrahydrofolsäure, Dihydrofolsäure, Folsäure, Tetrahydroneopterin, Dihydroneopierin, Neopterin, Methotrexat, Pterin, 6-Hydroxymethylpterin, Xanthopterin und Isoxanthopterin zeigten keine signifikanten Effekte. Nur mit 5-Deaza-6-methyl-5,6,7,8-tetrahydropterin konnte eine schwache Hemmung der NO-Synthase erreicht werden. Overfeld et al. (Br. J. Pharmacol., **107** (1992) 1008) beobachteten in intakten alveolaren Rattenmacrophagen eine Hemmung der NO-Produktion durch BH₄ und Sepiapterin, die vermutlich auf einem feed back-Mechanismus beruht. Pterin-6-carbonsäure zeigte in diesen Tests keine Wirkung.

Bömmel et al. (J. Biol. Chem. 273 (1998) 33142 und Portland Press Proc. 15 (1998) 57) verwendeten Pterine und photolabile Pterinderivate zur Charakterisierung der Tetrahydrobiopterin-Bindungsstelle der NO-Synthase.

Die Verwendung von Pteridinonen zur Hemmung der NO-Synthase wird in WO-A-94/14780 offengelegt. EP-A-0,760,818 und EP-A-0,760,664 beschreiben die Verwendung eine Reihe unterschiedlich substituierter Pteridine und Tetrahydropteridine zur Hemmung der NO-Synthase. Die darin beschriebenen Pterine und Pteridine sind jedoch hinsichtlich einiger Eigenschaften, wie Aktivität, Selektivität für bestimmt NO-Synthase-Isoformen und Löslichkeit weiterhin verbesserungsbedürftig.

Pfeiffer et al.(Biochem. J. 328 (1997) 349) beschreiben 4-Aminobiopterin als Hemmstoff der NO-Synthase (Biochem. J., 328 (1997) 349). Diese Verbindungen weisen u.a. eine freie Amino-Gruppe in 4-Position und eine im Vergleich zum natürlichen Cofaktor unveränderte Seitenkette in 6-Position auf. Kürzlich gelöste Röntgensdukturen (B. R. Crane et al., Science 279 (1998) 2121) zeigen Wechselwirkungen dieser Verbindungen mit NO-Synthase.

Überraschend wurde nun gefunden, daß insbesondere Pteridine, deren Aminogruppe in 4-Position durch Substituenten, bevorzugt durch Alkylierung bzw. Dialkylierung, weitgehend blockiert ist, und die in 6-Position eine vorwiegend lipophile Gruppe tragen, potente Hemmstoffe der NO-Synthase sind und als solche zur Behandlung von Krankheiten, die mit einem erhöhten NO-Spiegel in Verbindung stehen, verwendet werden können.

Die Pterine der allgemeinen Formel I stellen im Vergleich zu den in EP 0 760 818 und EP 0 760 664 offengelegten Pterinen vor allem in Hinblick auf die NO-Synthase hemmende Wirkung, Isoformselektivität und die nachhaltig verbesserten Löslichkeitseigenschaften einen erheblichen und in jeder Hinsicht überraschenden Fortschritt dar.

Gegenstand der vorliegenden Erfindung sind Verbindungen der Formeln (Ia), (Ib) und (Ic) 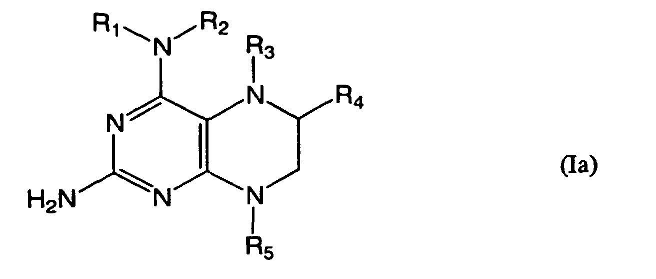 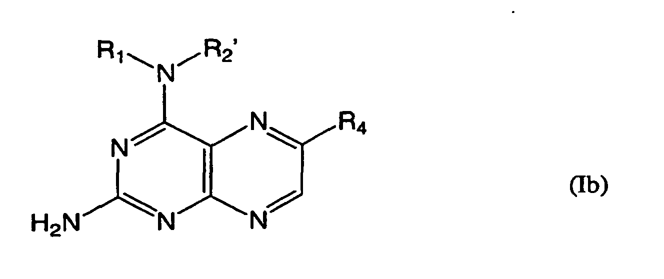 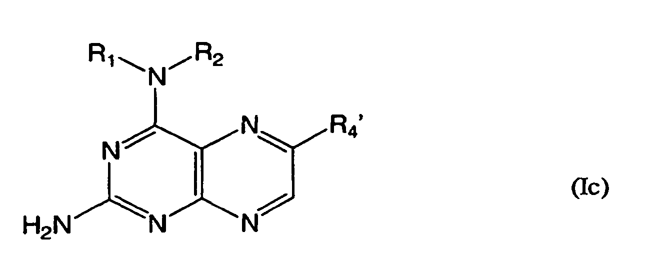 worin
- R¹: für Wasserstoff, C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl, Alkylaryl oder Arylalkyl steht, wobei die organischen Reste durch einen oder mehrere Substituenten R⁶ substituiert sein können,
- R²: unabhängig von R¹ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl oder Arylalkyl steht, wobei die organischen Reste durch einen oder mehrere Substituenten R⁶ substituiert sein können,
- R^{2'}: unabhängig von R¹ für C₂-C₂₀-Alkenyl, C₂-C₂₀-Alkinyl, Cycloalkenyl mit drei bis acht Ringkohlenstoffatomen, Cycloalkylalkyl mit fünf bis sechs Ringkohlenstoffatomen oder Heteroaryl steht, wobei die organischen Reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann, und der gegebenenfalls mit einem oder mehreren Resten R⁶ substituiert ist,
- R³: für Wasserstoff, -CO-Alkyl, -CO-Alkylaryl oder -CO-Aryl steht,
- R⁴: für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl, Arylalkyl, -CO-O-Alkyl, -CO-O-Aryl, -CO-Alkyl, -CO-Aryl steht, wobei die organischen Reste durch einen oder mehrere Substituenten R⁷ substituiert sein können,
- R^{4'}: für C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Heteroaryl oder -CO-O-Alkyl, -CO-O-Aryl, -CO-Alkyl, -CO-Aryl steht, wobei die organischen Reste durch einen oder mehrere Substituenten R⁷ substituiert sein können,
- R⁵: unabhängig von R³ für Wasserstoff, -CO-Alkyl, -CO-Alkylaryl oder -CO-Aryl steht,
- R⁶: für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹, -COOH, -CO-O-(C₁-C₅)-Alkyl, -CO-O-Aryl, -S(O)ₙ-(C₁-C₅)-Alkyl, -SO₂-NR⁸R⁹ steht,
- R⁷: unabhängig von R⁶ eine der Bedeutungen von R⁶ hat,
- R⁸: für Wasserstoff oder C₁-C₂₀-Alkyl steht,
- R⁹: für Wasserstoff, C₁-C₂₀-Alkyl oder Aryl steht,

in allen ihren stereoisomeren und tautomeren Formen und Mischungen davon in allen Verhältnissen, und ihre physiologisch verträglichen Salze, Hydrate, und Ester.

Gegenstand der vorliegenden Erfindung sind ferner Arzneimittel enthaltend eine Verbindung der allgemeinen Formel I 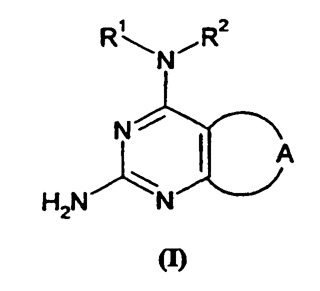 wobei
- A: für eine Verbrückung der Form 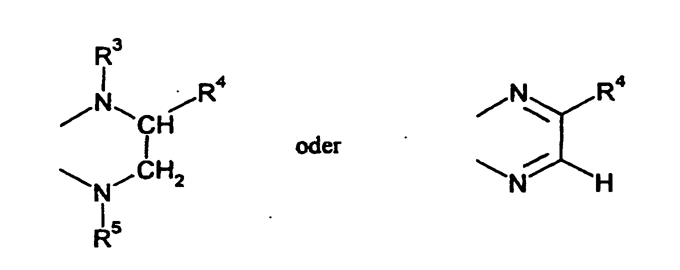
steht,
- R¹: für Wasserstoff, C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl, Alkylaryl oder Arylalkyl steht, wobei die organischen Reste durch einen oder mehrere Substituenten R⁶ substituiert sein können,
- R²: unabhängig von R¹ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl oder Arylalkyl steht, wobei die organischen Reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann, und der gegebenenfalls mit einem oder mehreren Resten R⁶ substituiert ist,
- R³: für Wasserstoff, -CO-Alkyl, -CO-Alkylaryl oder -CO-Aryl steht,
- R⁴: für C₁-C₂₀-Alkyl, C₂-C₂₀-Alkerlyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl, Arylalkyl, -CO-O-Alkyl, -CO-O-Aryl, -CO-Alkyl, -CO-Aryl steht, wobei die organischen Reste durch einen oder mehrere Substituenten R⁷ substituiert sein können,
- R⁵: unabhängig von R³ für Wasserstoff, -CO-Alkyl, -CO-Alkylaryl oder -CO-Aryl steht,
- R⁶: für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹, -COOH, -CO-O-(C₁-C₅)-Alkyl, -CO-O-Aryl, -S(O)ₙ-(C₁-C₅)-Alkyl, -SO₂-NR⁸R⁹,
- R⁷: unabhängig von R⁶ eine der Bedeutungen von R⁶ hat,
- R⁸: für Wasserstoff oder (C₁-C₂₀) Alkyl steht,
- R⁹: für Wasserstoff, (C₁-C₂₀) Alkyl oder Aryl steht,
in allen ihren stereoisomeren und tautomeren Formen und Mischungen davon in allen Verhältnissen, und ihre physiologisch verträglichen Salze, Hydrate, und Ester als Hemmstoff der NO-Synthase.

Können Gruppen oder Substituenten mehrfach in den Verbindungen der Formeln I, (Ia),(Ib) oder (Ic) vorkommen, so können sie alle unabhängig voneinander die angegebenen Bedeutungen haben und können jeweils gleich oder verschieden sein.

Alkylreste können geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind. Alkylreste enthalten üblicherweise ein bis zwanzig Kohlenstoffatome, vorzugsweise ein bis zehn Kohlenstoffatome.

Beispiele für Alkylgruppen sind Methyl, Ethyl, Propyl, Butyl, Pentyl, Hexyl, Heptyl, Octyl, Nonyl, Decyl, die n-Isomeren dieser Reste, Isopropyl, Isobutyl, Isopentyl, *sec*Butyl, tert-Butyl, Neopentyl, 3,3-Dimethylbutyl.

Beispiele für Alkenylreste sind geradkettige oder verzweigte Kohlenwasser-stoffreste, die eine oder mehrere Doppelbindungen enthalten. Alkenylreste enthalten üblicherweise zwei bis zwanzig Kohlenstoffatome und ein oder zwei Doppelbindungen, vorzugsweise zwei bis zehn Kohlenstoffatome und eine Doppelbindung.

Beispiele für Alkinylreste sind geradkettige oder verzweigte Kohlenwasserstoffreste, die eine oder mehrere Dreifachbindungen enthalten. Alkinylreste enthalten üblicherweise zwei bis zwanzig Kohlenstoffatome und ein oder zwei Dreifachbindungen, vorzugsweise zwei bis zehn Kohlenstoffatome und eine Dreifachbindung.

Beispiele für Alkenylreste sind der Vinylrest, der 2-Propenylrest (Allylrest), der 2-Butenylrest und der 2-Methyl-2-propenylrest.

Beispiele für Alkinylreste sind der Ethinylrest, der 2-Propinylrest (Propargylrest) oder der 3-Butinylrest.

Cycloalkylreste sind gesättigte cyclische Kohlenwasserstoffe, die üblicherweise drei bis acht Ring-Kohlenstoffatome enthalten, vorzugsweise fünf oder sechs Ring-Kohlenstoffatome. Cycloalkylreste können ihrerseits substituiert sein.

Beispiele für Cycloalkylreste sind Cyclopropyl, Cyclobutyl, Cyclopentyl, Cyclohexyl, Cycloheptyl und Cycloctyl, die alle auch zum Beispiel durch einen oder mehrere gleiche oder verschiedene (C₁-C₄)-Alkylreste, insbesondere durch Methyl, substituiert sein können. Beispiele für solche substituierten Cycloalkylreste sind 4-Methylcyclohexyl, oder 2,3-Dimethylcyclopentyl.

Cycloalkenylreste sind ungesättigte cyclische Kohlenwasserstoffe, die üblicherweise drei bis acht Ring-Kohlenstoffatome enthalten, vorzugsweise fünf oder sechs Ring-Kohlenstoffatome. Vorzugsweise weisen Cycloalkenylreste eine Doppelbindung im Ringsystem auf Cycloalkenylreste können ihrerseits substituiert sein.

Cycloalkylalkylreste sind gesättigte Kohlenwasserstoffe, die sich von einer Cycloalkylsubstituierten Alkylgruppe ableiten. Üblicherweise weist die Cycloalkylgruppe fünf bis sechs Ring-Kohlenstoffatome auf Beispiele für Cycloalkylalkylreste sind Cyclopentylmethyl, Cyclopentylethyl, Cyclohexylethyl und insbesondere Cyclohexylmethyl. Cycloalkylalkylreste können ihrerseits substituiert sein.

Aryl steht für einen carbocyclischen oder heterocyclischen aromatischen Rest, vorzugsweise für Phenyl, Naphtyl oder Heteroaryl. Arylreste können unsubstituiert oder substituiert sein. Substituenten sind ein oder mehrere gleiche oder verschiedene einwertige organische Reste, beispielsweise oder aus der Reihe Halogen, Alkyl, Phenyl, -OH, -O-Alkyl, Alkylendioxy, -NR⁸R⁹, -NO₂, -CO-(C₁-C₅)-Alkyl, -CF₃, -CN, -CONR⁸R⁹, -COOH, -CO-O-(C₁-C₅)-Alkyl, -S(O)ₙ-(C₁-C₅)-Alkyl, -SO₂-NR⁸R⁹.

Alkylaryl steht für einen alkylsubstituierten Arylrest, vorzugsweise für (C₁-C₃)-Alkylaryl, insbesondere Methylphenyl.

Arylalkyl steht für einen Arylsubstituierten Alkylrest, vorzugsweise für Phenylmethyl oder 2-Phenylethyl.

Heteroaryl oder ein heterocyclischer aromatischer Rest steht vorzugsweise für einen 5- bis 7-gliedrigen ungesättigten Heterocyclus, der ein oder mehrerer Heteroatome aus der Reihe O, N, S aufweist.

Beispiele für Heteroaryle von denen sich in den Verbindungen der Formeln I (Ia), (Ib) oder (Ic) vorkommende Reste ableiten können, sind Pyrrol, Furan, Thiophen, Imidazol, Pyrazol, 1,2,3-Triazol, 1,2,4-Triazol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin oder1,3-Thiazepin.

Die von diesen Heterocyclen abgeleiteten Reste können über jedes geeignete Kohlenstoffatom gebunden sein. Stickstoffheterocyclen, die an einem Ring-Stickstoffatom ein Wasserstoffatom (oder einen Substituenten) tragen, zum Beispiel Pyrrol, Imidazol, etc., können auch über ein Ring-Stickstoffatom gebunden sein, insbesondere, wenn der betreffende Stickstoffheterocyclus an ein Kohlenstoffatom gebunden ist. Ein Thienylrest kann beispielsweise als 2-Thienylrest oder 3-Thienylrest vorliegen, ein Furanrest als 2-Furylrest oder 3-Furylrest, ein Pyridylrest als 2-Pyridylrest, 3-Pyridylrest oder 4-Pyridylrest.

Halogen bedeutet Fluor, Chlor, Brom oder Iod, vorzugsweise Fluor oder Chlor.
- R¹: ist bevorzugt Wasserstoff, (C₂-C₄)-Alkyl, das durch einen oder mehrere Substituenten R⁶ substituiert sein kann, (C₁-C₂)-Alkylaryl, besonders bevorzugt steht R¹ für Arylmethyl
- R²: ist bevorzugt (C₂-C₄)-Alkyl, das durch einen oder mehrere Substituenten R⁶ substituiert sein kann, (C₁-C₂)-Alkylaryl, besonders bevorzugt steht R² für Arylmethyl
darüberhinaus bilden R¹ und R² bevorzugt zusammen mit dem sie tragenden Stickstoffatom einen 5-7-gliedrigen Ring, der vorzugsweise kein oder nur ein weiteres Heteroatom aus der Reihe N, O, S enthält. Ganz besonders bevorzugte Ringe dieser Art sind Pyrrolidin, Piperidin, Morpholin, Dimethylmorpholin, Thiomorpholin oder N-(C₁-C₂)-Alkylpiperazin, wobei diese Ringe selbst auch substituiert sein können, beispielsweise durch -OH, -O-(C₁-C₃)-Alkyl, -NR⁸R⁹ oder -COOH.
- R³: ist bevorzugt Wasserstoff, CO-(C₁-C₃)-Alkyl oder CO-Aryl, ganz besonders bevorzugt steht R³ für Wasserstoff
- R⁴: steht bevorzugt für Aryl, (C₁-C₅)-Alkyl, das durch einen oder mehrere Substituenten R⁷ substituiert sein kann, oder -CO-O-Aryl. Besonders bevorzugte Reste R⁴ sind Aryl und 1,2-Dihydroxypropyl.
- R⁵: steht bevorzugt für Wasserstoff.
- R⁶: steht bevorzugt für -OH, -O-(C₁-C₃)-Alkyl, -NR⁸R⁹ oder -COOH.
- R⁷: steht bevorzugt für -OH, -O-(C₁-C₁₀)-Alkyl, Phenoxy, Oxo, besonders bevorzugt für -OH, Decyloxy und Phenoxy.
- Aryl: ist bevorzugt Phenyl, Thiophenyl, Furyl und Pyridyl, besonders bevorzugt ist Phenyl die alle wie beschrieben substituiert sein können. Bevorzugte Substituenten sind (C₁-C₃)-Alkyl, Halogen und (C₁-C₃)-Alkyloxy und (C₁-C₂)-Alkylendioxy. Die bevorzugte Anzahl von Substituenten an Arylresten ist 0,1 oder 2; Phenylsubstituenten befinden sich vorzugsweise in der meta- oder para-Position, im Falle zweier Substituenten in der 3- und 4-Position.
- n: steht bevorzugt für 0 und 2

In Bezug auf sogenannte Struktur-Wirkungsbeziehungen ist festauhalten, daß hier insbesondere die 4- und die 6-Position des Pterin-Gerüsts von Bedeutung zu sein scheinen. Bei den Tetrahydropterinen (vergl. Formel (b)) erhöhen z.B. großvolumige Substituenten in 6-Position, wie z.B. substituiertes Phenyl, die Aktivität der Wirkstoffe. Bei den aromatischen Strukturen (vergl. Formel (a)) ist eine Aktivitätserhöhung bevorzugt dann zu beoachten, wenn der Aminosubstituent in 4-Position dialkylyliert bzw diaralkyliert ist und die 6-Position aryliert ist.

Die Erfindung umfaßt alle möglichen Enantiomeren und Diastereomere der Verbindungen der allgemeinen Formeln (Ia), (Ib) und (Ic) sowie der in Arneimitteln enthaltenen Verbindungen der allgemeinen Formel I, ebenso Mischungen von zwei oder mehr stereoisomeren Formen, zum Beispiel Mischungen von Enantiomeren und/oder Diastereomeren, in allen Verhältnissen.

Enantiomere werden also in enantiomerenreiner Form, sowohl als linksdrehende als auch als rechtsdrehende Antipoden, in Form von Racematen und in Form von Mischungen der beiden Enantiomeren in allen Verhältnissen von der Erfindung umfaßt. Bei Vorliegen einer cis/trans-Isomerie werden sowohl die cis-Form als auch die trans-Form und Gemische dieser Formen in allen Verhältnissen von der Erfindung umfaßt. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formeln (Ia), (Ib) und (Ic) sowie der in Arneimitteln enthaltenen Verbindungen der allgemeinen Formel I

Die Erfindung umfaßt auch die entsprechenden physiologisch oder toxikologisch verträglichen Salze, insbesondere die pharmazeutisch verwendbaren Salze. So können die Verbindungen der Formel, Formeln (Ia), (Ib) und (Ic) sowie der in Arneimitteln enthaltenen Verbindungen der allgemeinen Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren.

Verbindungen der, Formeln (Ia), (Ib) und (Ic) sowie der in Arzneimitteln enthaltenen Verbindungen der allgemeinen Formel I die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw.

Enthalten die Verbindungen der Formeln (Ia), (Ib) und (Ic) sowie der in Arzneimitteln enthaltenen Verbindungen der allgemeinen Formel I gleichzeitig saure und basische Gruppen im Molekül, so gehören neben den geschilderten Salzformen auch innere Salze oder Betaine (Zwitterionen) zu der Erfindung.

Salze können aus den Verbindungen der Formeln (Ia), (Ib) und (Ic) sowie der in Arzneimitteln enthaltenen Verbindungen der allgemeinen Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt weiterhin alle Solvate von Verbindungen der Formeln (Ia), (Ib) und (Ic) sowie der in Arzneimitteln enthaltenen Verbindungen der allgemeinen Formel I zum Beispiel Hydrate oder Addukte mit Alkoholen, sowie Derivate der Verbindungen der Formel I wie zum Beispiel Ester, und Pro-Drugs und aktive Metabolite.

Erfindungsgemäße Verbindungen der allgemeinen Formeln (Ia), (Ib) und (Ic) sowie der in Arzneimitteln enthaltenen Verbindungen der allgemeinen Formel I können gemäß folgendem Syntheseschema 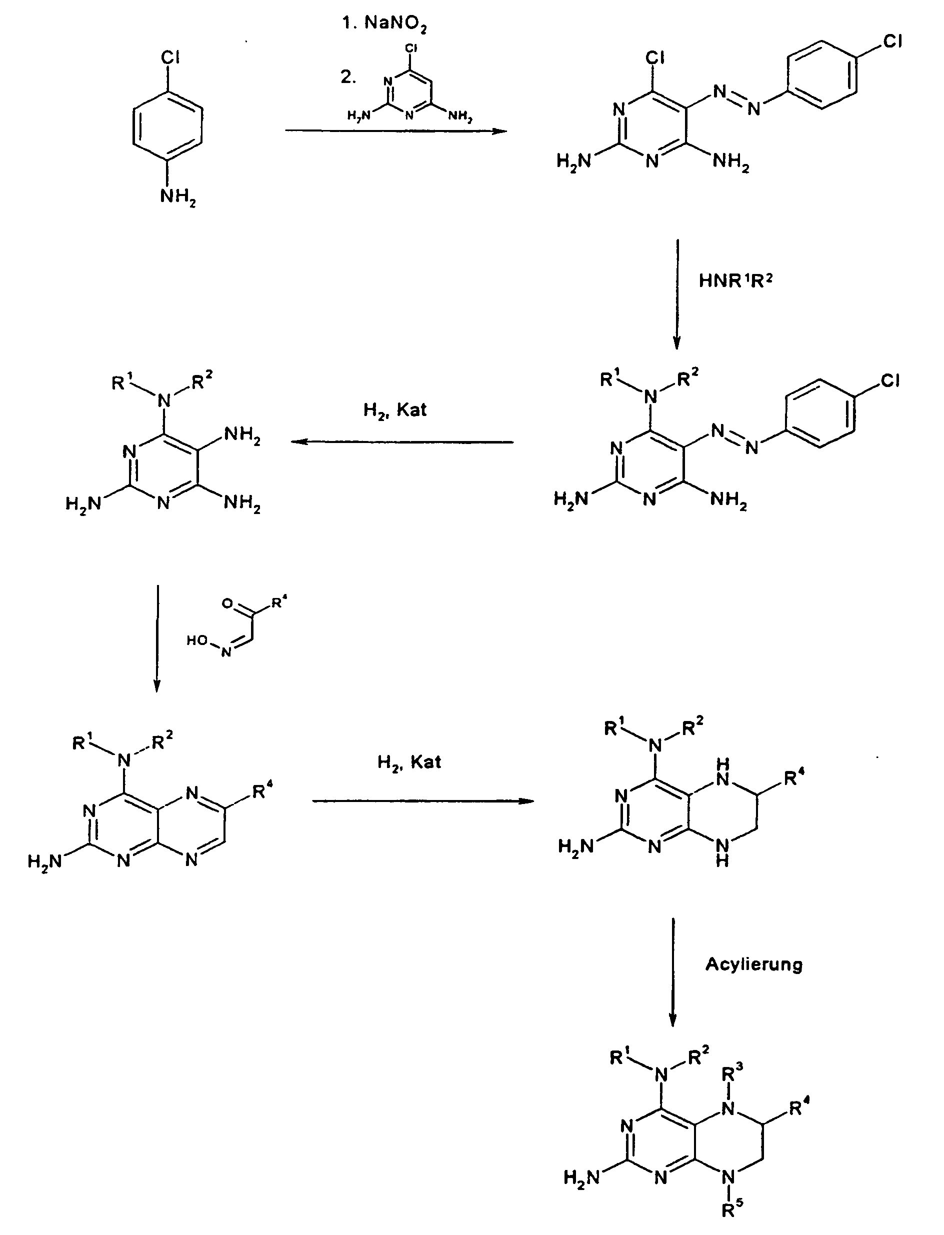 erhalten werden:

Das Schema wird nachfolgend näher erläutert:

Zur Synthese der Verbindungen der allgemeinen Formel I wird 2,6-Diamino-4-chlor-5-p-chlorphenylazopyrimidin (II) in Substanz oder in einem Lösungsmittel wie etwa DMF, Toluol oder Tetrahydrofuran mit einem 2-20-fachen Überschuß eines Amins der allgemeinen Formel HNR¹R² (III) bei einer Temperatur, die vorzugsweise zwischen Raumtemperatur (RT) und dem Siedepunkt des Lösungsmittels liegt, umgesetzt Alternativ kann die Umsetzung auch mit einer äquimolaren Menge des Amins in Gegenwart einer Hilfsbase wie etwa Triethylamin oder Hünig-Base vorgenommen werden.

Die resultierenden 2,6-Diamino-4-(subst.-amino)-5-p-chlorphenylazopyrimidine (IV) werden in einem Lösungsmittel wie etwa Methanol, Ethanol oder Wasser, vorzugsweise in Gegenwart einer Säure wie etwa HCl oder Essigsäure, oder in Gegenwart einer Base, wie etwa Ammoniak, mit Hilfe eines Katalysators wie etwa Raney-Nickel, Platindioxid oder Palladium auf Kohle bei einem Wasserstoffdruck zwischen 1 und 200atm hydriert.

Die auf diese Weise erhaltenen 2,5,6-Triamino-4-(subst.-amino)-pyrimidine (V) werden sodann in einem Lösungsmittel wie etwa Methanol, Ethanol, DMF oder Wasser mit dem jeweiligen, den Rest R⁴ enthaltenden Glyoxalmonoxim (VI) vermischt und diese Mischung bis zur vollständigen Umsetzung bei einer Temperatur, die zwischen RT und dem Siedepunkt des eingesetzten Lösungsmittels liegt gerührt. Nach dem Abkühlen wird die Suspension oder Lösung mit einer Base wie etwa Ammoniak basisch gestellt, der ausgefallene Niederschlag abgesaugt, mit Wasser gewaschen und getrocknet.

Eine Lösung des resultierenden Pteridins wird in einem Lösungsmittel wie THF, Methanol oder Ethanol unter Zuhilfenahme eines Katalysators wie etwa Raney-Nickel, Platindioxid oder Palladium auf Kohle bei einem Wasserstoffdruck zwischen 1 und 200atm hydriert.

Die weitere Derivatisierung zur Einführung der Substituenten R³ und/oder R⁴ kann nach den dem Fachmann bekannten Standardverfahren für Acylierungen vorgenommen werden.

Die o.g. Reaktionen zur Herstellung von 4-Aminopteridinderivaten sind grundsätzlich beispielsweise in WO-A-97/21711 beschrieben.

Das o.g. Verfahren zur Herstellung der Verbindungen der Formel I ist ebenfalls Gegenstand der vorliegenden Erfindung.

Die Verbindungen der Formel V werden dadurch ebenfalls in der vorliegenden Erfindung verwendet.

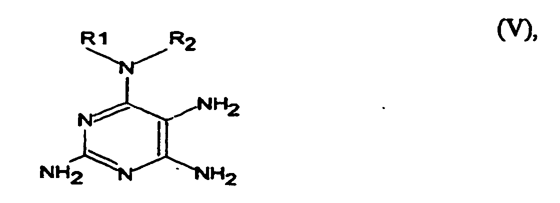

Darin besitzen R¹ und R² die weiter oben definierte Bedeutung.

Krankheiten, die durch einen erhöhten NO-Spiegel entstehen und die somit erfindungsgemäß mit Verbindungen der allgemeinen Formeln I, (Ia), (Ib) oder (Ic) behandelt werden können bzw. denen mit diesen vorgebeugt werden kann, sind insbesondere pathologische Blutdruckabfälle, wie sie beim septischen oder hämorrhagischen Schock, bei der Tumortherapie mit Cytokinen oder bei der Leberzirrhose auftreten oder Autoimmunerkrankungen, wie Typ I Diabetes sowie Atherosklerose. Desweiteren entzündliche Erkrankungen, wie rheumatoide Arthritis und insbesondere Colitis ulcerosa, sowie insulinabhängige Diabetes mellitus und Transplantatabstoßungsreaktionen.

Auch die folgenden Erkrankungen stehen im Zusammenhang mit einer gesteigerten Produkton von Stickstoffmonoxid und können erfindungsgemäß behandelt werden. Im Bereich Herz-Kreislauf sind dies Atherosklerose, postischämische Reperfusionsschäden, Myokarditis auf der Grundlage einer Coxsackie-Virus-Infekton und Kardiomypathie; im Bereich Zentrales Nervensystem Neuritisformen, Enzephalomyelitiden, virale neurodegenerative Erkrankungen, Morbus Alzheimer, Hyperalgesie, Epilepsie und Migräne; im Bereich Niere akutes Nierenversagen sowie Glomerulonephritis.

Außerdem sind auch Behandlungen im Bereich des Magens und des Uterus/Plazenta sowie eine Beeinflussung der Motalität der Spermien Anwendungsgebiete für Verbindungen der allgemeinen Formeln I, (Ia), (Ib) oder (Ic).

Die Verbindungen der Formeln I, (Ia), (Ib) oder (Ic) und ihre physiologisch verträglichen Salze, Hydrate, Ester und Addukte können somit am Tier, bevorzugt am Säugetier, und insbesondere am Menschen als Arzneimittel für sich allein, in Mischungen untereinander oder zusammen mit anderen Wirkstoffen verwendet werden. Gegenstand der vorliegenden Erfindung ist daher insbesondere auch die Verwendung von Verbindungen der Formel I und ihren physiologisch verträglichen Salzen, Hydraten und Estern zur Herstellung eines Medikaments zur Therapie oder Prophylaxe der vorstehend genannten Krankheitsbilder sowie die Verwendung zur Herstellung eines Medikaments zur Absenkung oder Normalisierung eines NO-Spiegels.

Ebenso ist Gegenstand der Erfindung die Verwendung der Verbindungen der Formel I und ihrer physiologisch verträglichen Salze, Hydrate und Ester zur Hemmung der NO-Synthase, ihre Verwendung zur Therapie oder Prophylaxe der vorstehend genannten Krankheitsbilder und ihre Verwendung zur Normalisierung eines gestörten NO-Haushalts.

Ebenso umfaßt sind Arzneimittel, die die Verbindungen der Formel I, ihre physiologisch verträglichen Salze, Ester und Hydrate und Ester allein, in Mischungen untereinander oder zusammen mit anderen Wirkstoffen neben üblichen Hilfs- und Zusatzstoffen enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbocromen; Beruhigungsmittel, wie z.B. Barbituresäurederivate, 1,4-Benzodiazepine und Meprobamat; Diuretica, wie z.B. Clororthiazid; das Herz tonisierende Mittel, wie z.B. Digitalispräparate; blutdrucksenkende Mittel, wie z.B. Hydralazin, Dihydralazin, ACE-Hemmer, Prazosin, Clonidin, Rauwolfia-Alkaloide; Mittel, die den Fettsäurespeigel im Blut senken, wie z.B. Bezafibrat; Fenofibrat; Mittel für die Thromboseprophylaxe, wie z.B. Phenprocoumon; entzündungshemmende Substanzen, wie etwa Corticosteroide; Salicylate, oder Propionsäure-derivate, wie beispielsweise Ibuprofen; Antibiotika, wie z.B. Penicilline oder Cephalosporine; NO-Donoren, wie z.B. organische Nitrate oder Sydnonimine.

Arzneimittel gemäß der vorliegenden Erfindung können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die Herstellung der erfindungsgemäßen Medikamente kann nach den bekannten Standardverfahren zur Herstellung pharmazeutischer Präparate erfolgen.

Dazu werden ein oder mehrere Verbindungen der Formeln I, (Ia), (Ib) oder (Ic) und/oder ihre physiologisch verträglichen Salze, Ester und Hydrate zusammen mit einem oder mehreren festen oder flüssigen galenischen Trägerstoffen und/oder Zusatzstoffen oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann. Die pharmazeutischen Präparate enthalten eine therapeutisch oder prophylaktisch wirksame Dosis der Verbindungen der Formeln I, (Ia), (Ib) oder (Ic) und/oder ihrer physiologisch verträglichen Salze, Ester und Hydrate, die normalerweise 0.5 bis 90 Gewichtsprozent des pharmazeutischen Präparats ausmacht.

Für die Herstellung beispielsweise von Pillen, Tabletten, Dragees und Hartgelatinekapsein kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze, Ester und Hydrate können auch lyophilisiert werden und die erhaltenen Lyophilisate zum Beispiel zur Herstellung von Injektionspräparaten oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien.

Die Dosierung des zu verabreichenden Wirkstoffs der Formeln I, (Ia),(Ib) oder (Ic) und/oder eines physiologisch verträglichen Salzes, Esters oder Hydrats davon, hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formeln I, (Ia),(Ib) oder (Ic) weitere Wirkstoffe verabreicht werden. Im allgemeinen ist eine Tagesdosis von etwa 0.01 bis 100 mg/kg, vorzugsweise 0. 1 bis 10 mg/kg, insbesondere 0.3 bis 5 mg/kg (jeweils mg pro kg Körpergewicht) bei Verabreichung an einen ca. 75 kg schweren Erwachsenen zur Erzielung der angestrebten Wirkung angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 200 mg Wirkstoff der Formeln I, (Ia),(Ib) oder (Ic) und/oder dessen physiologisch verträgliche Salze.

Die Verbindungen der Formeln I, (Ia),(Ib) oder (Ic) hemmen die verschiedenen Isoformen der NO-Synthase vornehmlich durch Bindung in der Tetrahydrobiopterin-Bindungstasche des Enzyms. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Hemmung der NO-Synthase beabsichtigt ist, sowie für diagnostische Zwecke, zum Beispiel in der in vitro-Diagnostik von Zellproben oder Gewebsproben. Ferner können die Verbindungen der Formeln I, (Ia),(Ib) oder (Ic) und ihre Salze, Ester oder Hydrate als Zwischenprodukte zur Herstellung weiterer Arzneimittelwirkstoffe dienen.

### Beispiele

### Nachfolgende Herstellvorschriften und Beispiele erläutern die Erfindung, ohne sie einzuschränken:

### 2,6-Diamino-4-chloro-5-p-chlorophenylazopyrimidin

Eine Lösung aus p-Chloranilin (25.5 g, 0.2 moles) in 6 N HCl (100 mL) wurde auf 0-5°C abgekühlt und anschließend eine Lösung aus NaNO₂ (13.8 g, 0.2moles) in Wasser (40 ml) unter Rühren zugetropft. Nach Beendigung der Zugabe, wurde die Lösung weitere 15 min gerührt und der Reaktionsverlauf mit Hilfe von Jodstärkepapier (Blaufärbung) überprüft. Durch Zugabe von Harnstoff (5 g) wurde der Überschuss an HNO₂ zerstört. Die Diazonium-Salz-Lösung wurde in eine Lösung von 2,6-Diamino-4-chlorpyrimidin (26.0 g, 0.18 moles) in Wasser (500 mL) gegossen und für 30 min gerührt. Anschließend wurde Kaliumacetat (70 g) zugegeben und die Mischung wurde für 16 Stunden bei Raumtemperatur gerührt. Das ausgefallene Produkt wurde abgesaugt, mit H₂O gewaschen und im Exsikkator unter Vakuum über P₄O₁₀ getrocknet. Ausbeute: 44.0 g (81%) gelber Feststoff. Umkristallisation aus DMF/H₂O. Smp.: 268°C.

### 2,6-Diamino-4-alkylamino-5-p-chlorphenylazopyrimidine

### Allgemeine Arbeitsvorschrift:

Eine Lösung von 2,6-Diamino-4-chlor-5-p-chlorphenylazopyrimidin (5.0 g, 16.6 mmol) und 10g des Amins in DMF (50 mL) wurde in einem Ölbad bei 70°C für 5 Stunden gerührt. Nach Zugabe von Wasser (50 mL) wurde abgekühlt und der Niederschlag abgesaugt, mit Wasser gewaschen, getrocknet und aus EtOH oder Aceton/Wasser umkristallisiert.

### Auf diese Weise wurden erhalten:

1.) 2,6-Diamino-4-diethylamino-5-p-chlorphenylazopyrimidin, Smp.: 145-148°C
2.) 2,6-Diamino-4-dibenzylamino-5-p-chlorphenylazopyrimidin, Smp.: 185-186°C.
3.) 2,6-Diamino-4-(morpholin-4-yl)-5-p-chlorphenylazopyrimidin, Smp.: 219-221°C.
4.) 2,6-Diamino-4-(piperidin-1-yl)-5-p-chlorphenylazopyrimidin, Smp.: 199-201°C.
5.) 2,6-Diamino-4-(4-methylpiperazin-1-yl)-5-p-chlorphenylazopyrimidin, Smp.: 218-220°C.

### 2,5,6-Triamino-4-alkylaminopyrimidine (Hydrochloride)

### Allgemeine Arbeitsvorschrift:

Eine Lösung von 10 mmol des 2,6-Diamino-4-alkylamino-5-p-chlorphenylazopyrimidins in Methanol (70 mL) und konz. Ammoniak (10 mL) wurde unter H₂-Atmosphäre in Gegenwart des Katalysators Raney-Nickel (3.5 g) für 2 Tage in einer Schüttelapparatur reduziert. Der Katalysator wurde unter Argon-Atmosphäre abfiltriert, und das Filtrat unter Vakuum bis zur Trockene eingedampft. Der Rückstand wurde zur Entfernung des p-Chloroanilins mit Ether behandelt und der verbleibende Feststoff über Nacht mit methanolischer HCl (10%, 50 mL) gerührt. Das Dihydrochlorid Salz wurde abgesaugt und unter Vakuum im Exsikkator über KOH getrocknet.

Auf diese Weise wurden erhalten:
6.) 2,5,6-Triamino-4-diethylaminopyrimidin dihydrochlorid, Smp.: 138-142°C
7.) 2,5,6-Triamino-4-dibenzylaminopyrimidin dihydrochlorid, Smp.: 165-167°C
8.) 2,5,6-Triamino-4-(morpholin-4-yl)-pyrimidin dihydrochlorid, Smp.: 215-218°C (Zersetzung)
9.) 2,5,6-Triamino-4-(piperidin-1-yl)-pyrimidin dihydrochlorid, Smp.: 238-242°C
10.) 2,5,6-Triamino-4-(4-methylpiperazin-1-yl)-pyrimidin trihydrochlorid, Smp.: 226-230°C (Zersetzung)

### 2-Amino-4-alkylamino-6-(R⁴)-pteridine

### Allgemeine Arbeitsvorschrift:

Zu einer kochenden Lösung aus 2,5,6-Triamino-4-alkylaminopyrimidin-dihydrochlorid salz (5 mmol) in MeOH (20 mL) wurde eine Lösung aus des den Rest R⁴ enthaltenden Arylglyoxalmonoxims (7.5 mmol) in MeOH (10 mL) zugetropft und diese Mischung für 3 Stunden unter Rückfluß gekocht. Nach dem Abkühlen wurde die Suspension oder Lösung mit konz. Ammoniak auf pH 9-10 gestellt, der ausgefallene Niederschlag abgesaugt, mit Wasser gewaschen und getrocknet. Das Rohprodukt wurde aus aus EtOH und DMF/H₂O umkristallisiert.

Auf diese Weise wurden erhalten:
11.) 2-Amino-4-(dimethylamino)-6-phenyl-pteridin, Smp.: 247-250°C
12.) 2-Amino-4-(dimethylamino)-6-(4-methylphenyl)-pteridin, Smp.: 251-256°C
13.) 2-Amino-4-(dimethylamino)-6-(4-methoxyphenyl)-pteridin, Smp.: 280-284°C (Zersetzung)
14.) 2-Amino-4-(dimethylamino)-6-methoxymethyl-pteridin, Smp.: 237-239°C
15.) 2-Amino-4-(diethylamino)-6-phenyl-pteridin Hydrat, Smp.: 203-205°C
16.) 2-Amino-4-(diethylamino)-6-(4-chlorphenyl)-pteridin Dihydrat, Smp.: 250-254°C (Zersetzung)
17.) 2-Amino-4-(diethylamino)-6-(4-methoxyphenyl)-pteridin Hydrat, Smp.: 220-222°C
18.) 2-Amino-4-(diethylamino)-6-(3,4-dimethoxyphenyl)-pteridin Hydrat, Smp.: 182-185°C
19.) 2-Amino-4-(dibenzylamino)-6-phenyl-pteridin Dihydrat, Smp.: 225-227°C
20.) 2-Amino-4-(dibenzylamino)-6-(4-chlorophenyl)-pteridin Dihydrat, Smp.: 250-253°C
21.) 2-Amino-4-(dibenzylamino)-6-(4-methoxyphenyl)-pteridin, Smp.: 245-247°C
22.) 2-Amino-4-(dibenzylamino)-6-(3,4-dimethoxyphenyl)-pteridin Halbhydrat, Smp.: 200-201°C
23.) 2-Amino-4-(di-n-propylamino)-6-phenyl-pteridin Trihydrat, Smp.: 177-178°C
24.) 2-Amino-4-(di-n-propylamino)-6-(4-chlorophenyl)-pteridin Trihydrat, Smp.: 189-192°C (Zersetzung)
25.) 2-Amino-4-(di-n-propylamino)-6-(4-methoxyphenyl)-pteridin Hydrat, Smp.: 207-210°C (Zersetzung)
26.) 2-Amino-4-(di-n-propylamino)-6-(3,4-dimethoxyphenyl)-pteridin Hydrat, Smp.: 158-160°C
27.) 2-Amino-4-(morpholin-4-yl)-6-phenyl-pteridin Hydrat, Smp.: 224-227°C (Zersetzung)
28.) 2-Amino-4-(morpholin-4-yl)-6-(4-chlorophenyl)-pteridin Hydrochlorid Hydrat, Smp.: 252-254°C (Zersetzung)
29.) 2-Amino-4-(morpholin-4-yl)-6-(4-methoxyphenyl)-pteridin Hydrochlorid Hydrat, Smp.:238-240°C (Zersetzung)
30.) 2-Amino-4-(morpholin-4-yl)-6-(3,4-dimethoxyphenyl)-pteridin Trihydrat, Smp.: 218-220°C (Zersetzung)
31.) 2-Amino-4-(piperidin-1-yl)-6-phenyl-pteridin Dihydrat, Smp.: 209-211°C
32.) 2-Amino-4-(piperidin-1-yl)-6-(4-chlorophenyl)-pteridin Dihydrat, Smp.: 245-247°C (Zersetzung)
33.) 2-Amino-4-(piperidin-1-yl)-6-(4-methoxyphenyl)-pteridin Hydrat, Smp.: 211-214°C (Zersetzung)
34.) 2-Amino-4-(piperidin-1-yl)-6-(3,4-dimethoxyphenyl)-pteridin Hydrochlorid Dihydrat, Smp.:238-241°C (Zersetzung)
35.) 2-Amino-4-(4-methyl-piperazin-1-yl)-6-phenyl-pteridin Halbhydrat, Smp.: 245-247°C (Zersetzung)
36.) 2-Amino-4-(4-methyl-piperazin-1-yl)-6-(4-chlorophenyl)-pteridin Halbhydrat, Smp.: 277-279°C (Zersetzung)
37.) 2-Amino-4-(4-methyl-piperazin-1-yl)-6-(4-methoxyphenyl)-pteridin Halbhydrat, Smp.: 228-230°C (Zersetzung)
38.) 2-Amino-4-(4-methyl-piperazin-1-yl)-6-(3,4-dimethoxyphenyl)-pteridin Dihydrat, Smp.: 148-151°C (Zersetzung)
39.) 2-Amino-4-(pyrrolidin-1-yl)-6-(4-methoxyphenyl)-pteridin Dihydrat, Smp.: 243-246°C (Zersetzung)

### 2-Amino-4-alkylamino-6-(R⁴)-5,6,7,8-tetrahydropteridine

Allgemeine Arbeitsvorschrift:

Eine Lösung von zu reduzierenden Pteridins (3 mmol) in THF (25 ml) wurde mit PtO₂ (0.10 g)/H₂ katalytisch in einer Schüttelapparatur bewegt, bis die Wasserstoffaufnahme beendet war. Der Katalysator wurde abfiltriert, das Filtrat bis zur Trockne eingeengt und der Rückstand für mehrere Stunden unter Rühren mit methanolischer HCl behandelt. Die sich bildenden Kristalle wurden abgesaugt, mit Ether gewaschen und im Exsikkator unter Vakuum getrocknet.

Auf diese Weise wurden erhalten:
40.) 2-Amino-4-(morpholin-4-yl)-6-(4-methoxyphenyl)-5,6,7,8-tetrahydropteridin Hydrochlorid Halbhydrat, Smp.: 219-222°C
41.) 2-Amino-4-(morpholin-4-yl)-6-(3,4-dimethoxyphenyl)-5,6,7,8-tetrahydropteridin Hydrochlorid Hydrat, Smp.: 168°C
42.) 2-Amino-4-(morpholin-4-yl)-6-phenyl-5,6,7,8-tetrahydropteridin Dihydrochlorid Halbhydrat, Smp.: 200-203°C
43.) 2-Amino-4-(piperidin-1-yl)-6-(4-chlorophenyl)-5,6,7,8-tetrahydropteridin Trihydrochlorid Hydrat, Smp.: 170°C
44.) 2-Amino-4-(piperidin-1-yl)-6-(4-methoxyphenyl)-5,6,7,8-tetrahydropteridin Trihydrochlorid Hydrat, Smp.: 218-220°C
45.) 2-Amino-4-(piperidin-1-yl)-6-phenyl-5,6,7,8-tetrahydropteridin Dihydrochlorid Dihydrat, Smp.: 178-182°C
46.) 2-Amino-4-(di-n-propylamino)-6-phenyl-5,6,7,8-tetrahydropteridin Trihydrochlorid Hydrat, Smp.: 115°C
47.) 2-Amino-4-(di-n-propylamino)-6-(4-methoxyphenyl)-5,6,7,8-tetrahydropteridin Dihydrochlorid Dihydrat, Smp.: 120°C
48.) 2-Amino-4-(diethyllamino)-6-(4-chlorophenyl)-5,6,7,8-tetrahydropteridin Dihydrochlorid Semihydrat, Smp.: 138°C
49.) 2-Amino-4-(cyclohexylmethylamino)-6-(4-chlorophenyl)-5,6,7,8-tetrahydropteridin Dihydrochlorid Hydrat, Smp.: 160°C

Die Hemmung der Aktivität gereinigter Stickstoffmonoxid-Synthase (NOS) durch Verbindungen der allgemeinen Formel I kann wie folgt bestimmt werden.

Bei diesem Aktivitätsassay wird das bei der Bildung von NO durch gereinigte NOS anfallende Koprodukt L-Citrullin quantitativ erfaßt. Als Substrat der Enzymreaktion wird 3H-radiomarkiertes L-Arginin eingesetzt, das zu 3H-LCitrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillation gemessene 3H-Aktivität entspricht dann der Menge an L-Citrullin, die ein direktes Maß für die Aktivität der NOS ist.

Das Grundmedium für die Durchführung der Enzymreaktion ist TE-Puffer (Triethanolamin, EDTA, pH 7,0).

Das Endvolumen jeder Inkubation beträgt 100 gl. Das Reaktionsgemisch wird erhalten, indem die folgenden 6 Komponenten auf Eis gemischt werden:
1. "REA-Mix" (pH 7,0), der Triethanolamin, Calciumchlorid, Magnesiumchlorid, EDTA, L-Arginin, Calmodulin und Flavin-Adenin-Dinukleotid (FAD) enthält;
2. frisch zubereitete Stammlösung von β-Nicotinamid-Adenin-Dinukleotid-Phosphat, reduzierte Form (NADPH);
3. (6R)-5,6,7,8-Tetrahydro-L-Biopterin-Dihydrochlorid Stammlösung (BH₄) oder - für Versuche ohne BH₄ - stattdessen TE-Puffer;
4. gereinigte NO-Synthase aus Schweinekleinhirn oder aus Schweineleber;
5. L-[2,3,4,5-³H]-Arginin-Hydrochlorid-Stammlösung (1,52,6 TBq/mmol);
6. zu testende Substanz.

Die finalen Konzentrationen der Komponenten im Inkubationsvolumen von 100 gl sind: Triethanolamin 50 mM, EDTA 0,5 mM, CaCl₂ 226 µM, MgCl₂ 477 µM, L-Arginin 50 µM, Calmodulin 0,5 µM, FAD 5 µM, NADPH 1 mM, BH₄ (wenn zugesetzt) 2 µM, zu testende Substanz 100 µM.

Nach dem Mischen der Komponenten auf Eis wurde der Reaktionsansatz sofort in einem Wasserbad bei 37°C für 15 Minuten inkubiert. Zur Bestimmung der IC₅₀-Werte wurde in Gegenwart von 5kU/ml Catalase und für 45 Minuten inkubiert. Nach dieser Inkubationszeit wurde die Reaktion durch Zugabe von 900 gl eiskaltem "Stoppufier" (20 mM Natriumacetat, 2 mM EDTA, pH 5,5) abgestoppt und der Ansatz (Gesamtvolumen jetzt 1,0 ml) auf Eis gestellt. Zur Abtrennung des nicht umgesetzten 3H-L-Arginins wurde das Gemisch auf eine Ionenaustauschersäule mit 0,8 ml Dowex AG 50 WX-8 (100-200 mesh) gegeben, die zuvor mit 2 ml Stoppuffer gespült und äquilibriert wurde. Nach dem Auftragen der Probe wurde die Säule zweimal mit je 1 ml Wasser eluiert. Der Durchlauf der Probe und das Eluat wurden in Szintillationsgefäßen aufgefangen und gereinigt (Gesamtvolumen 3 ml). Zu den 3 ml wäßriger Meßlösung wurden 9 ml Szintillator-Lösung gegeben und die homogene Mischung wurde in einem Flüssigszintillationszähler Tricarb 2500 TR (Packard) 1 Minute pro Probe gemessen. Die mit der zu testenden Substanz gefundene Aktivität wurde in Prozent der Aktivität der Kontrolle angegeben. Jede Substanz wurde in einer Konzentration von 100 µM in Anwesenheit von 2 µM Tetrahydrobiopterin auf antagonistische Wirkung sowie in Abwesenheit von Tetrahydrobiopterin auf agonistische Wirkung auf die NOS getestet.

Alle Inkubationen wurden in Triplikaten angesetzt. Jeder Versuch wurde dreimal mit verschiedenen Enzympräparationen wiederholt. Einige Ergebnisse sind in der folgenden Tabelle 1 angegeben.

**Tabelle 1**

| **Beispiel** | **Restaktivität (% of V**_{**max**}**)** | **IC**_{**50**} **(µM)** |
|---|---|---|
| 11 | 92±11 | - |
| 12 | 15±7 | 75 |
| 13 | 13± 4 | 74 |
| 15 | 75± 3 | - |
| 16 | 42±10 | - |
| 17 | 2± 0.1 | 45 |
| 18 | 23±4 | - |
| 19 | 0± 0.05 | 3 |
| 20 | 0 | 3,5 |
| 21 | 0± 0.05 | 5 |
| 22 | 0 | 2 |
| 23 | 77±16 | - |
| 24 | 7±4 | - |
| 25 | 25±12 | - |
| 26 | 0 | 39 |
| 27 | 41± 8 | 82 |
| 28 | 3±1,5 | - |
| 29 | 5± 0.1 | 34 |
| 30 | 5±3 | - |
| 31 | 0± 0.05 | 62 |
| 32 | 3±1 | - |
| 33 | 7± 0.2 | 50 |
| 34 | 0 | 44 |
| 35 | 83± 1 | - |
| 36 | 64±9 | - |
| 37 | 84± 5 | - |
| 38 | 99±16 | - |
| 39 | 30±5 | - |
| 40 | 66±14 | - |
| 41 | 68±11 | - |
| 42 | 51±3 | - |
| 43 | 0 | 13 |
| 44 | 0 | 42 |
| 45 | 0 | 6 |
| 46 | 8±0.05 | - |
| 47 | 0 | 34 |
| 48 | 0 | 8 |
| 49 | 0 | 5 |

Ferner wurden die relativen Selektivitäten der Antipterin-Hemmstoffe auf die drei bekannten humanen NOS-Isoformen gemessen. Dabei wurden die IC₅₀-Werte für NOS-II/NOS-I und NOS-III/NOS-I gebildet (vergl. Tabelle 2).

Die Daten zeigen, daß die Substanzen für die Hemmung von NOS-I eine erhöhte Selektivität gegenüber NOS-II und eine erhöhte Selektivität gegenüber NOS-III aufweisen.

**Tabelle 2**

| **Substanz Beispiel** | **NOS Isoform** | **Aktivität (% von Kontrolle)** | **IC**_{**50**} **(µM)** | **Verhältnis NOS-II/I** | **Verhältnis NOS-III/I** |
|---|---|---|---|---|---|
| | NOS-I | 27 ± 1 | 18.7 | 21.3 | 5.3 |
| 21 | NOS-II | 81 ± 6 | 400^{a} | | |
| | NOS-III | 68 ± 2 | 100 | | |
| | NOS-I | 0 ± 0 | 22.0 | 3.7 | 0.6 |
| 45 | NOS-II | 50 ± 2 | 81.5 | | |
| | NOS-III | 11 ± 1 | 14.2 | | |
| | NOS-I | 1 ± 0,1 | 18,7 | 10.7 | 2.9 |
| 46 | NOS-II | 68 ± 1 | 200^{a} | | |
| | NOS-III | 27 ± 0,2 | 53.4 | | |
| | NOS-I | 0 ± 0,1 | 7,4 | 33.8 | 8.6 |
| 47 | NOS-II | 78 ± 0,4 | 250^{a} | | |
| | NOS-III | 31 ± 3 | 63.6 | | |
| | NOS-I | 2 ± 0 | 41.5 | 7.2 | 1.1 |
| 48 | NOS-II | 74 ± 4 | 300^{a} | | |
| | NOS-III | 27 ± 1 | 45.4 | | |
| | NOS-I | 0 ± 0,1 | 4,9 | 40.8 | 7.3 |
| 49 | NOS-II | 78 ± 6 | 200^{a} | | |
| | NOS-III | 18 ± 1 | 36 | | |

| | | | | | |
|---|---|---|---|---|---|
| ^{a} Bis zu 300 µM keine vollständige Enzymhemmung (IC₅₀ Werte extrapoliert). | | | | | |

## Patentansprüche

1. Verbindungen der Formeln (Ia), (Ib) und (Ic) 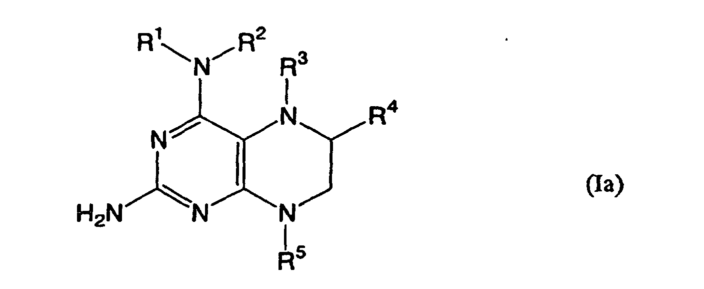 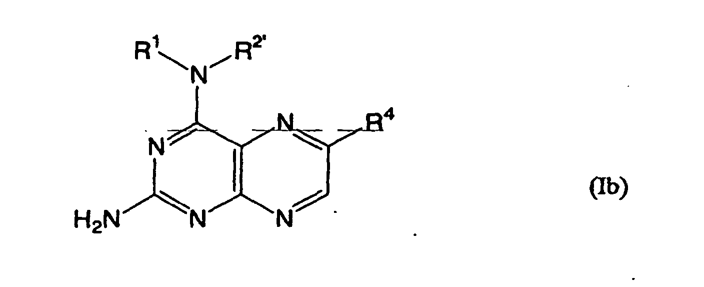 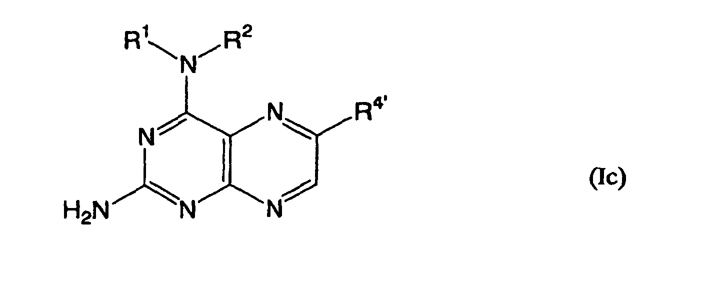 worin
R¹ für Wasserstoff, C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl, Alkylaryl oder Arylalkyl steht, wobei die organischen Reste durch einen oder mehrere Substituenten R⁶ substituiert sein können,
R² unabhängig von R¹ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl oder Arylalkyl steht, wobei die organischen Reste durch einen oder mehrere Substituenten R⁶ substituiert sein können,
R^{2'} unabhängig von R¹ für C₂-C₂₀-Alkenyl, C₂-C₂₀-Alkinyl, Cycloalkenyl mit drei bis acht Ringkohlenstoffatomen, Cycloalkylalkyl mit fünf bis sechs Ringkohlenstoff-atomen oder Heteroaryl steht, wobei die organischen Reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann, und der gegebenenfalls mit einem oder mehreren Resten R⁶ substituiert ist,
R³ für Wasserstoff, -CO-Alkyl, -CO-Alkylaryl oder -CO-Aryl steht,
R⁴ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl, Arylalkyl, -CO-O-Alkyl, -CO-O-Aryl, -CO-Alkyl, -CO-Aryl steht, wobei die organischen Reste durch einen oder mehrere Substituenten R⁷ substituiert sein können,
R^{4'} für C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Heteroaryl oder -CO-O-Alkyl, -CO-O-Aryl, -CO-Alkyl, -CO-Aryl steht, wobei die organischen Reste durch einen oder mehrere Substituenten R⁷ substituiert sein können,
R⁵ unabhängig von R³ für Wasserstoff, -CO-Alkyl, -CO-Alkylaryl oder -CO-Aryl steht,
R⁶ für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹, COOH, -CO-O-(C₁-C₅)-Alkyl, -CO-O-Aryl, -S(O)ₙ-(C₁-C₅)-Alkyl, -SO₂- NR⁸R⁹ steht,
R⁷ unabhängig von R⁶ eine der Bedeutungen von R⁶ hat,
R⁸ für Wasserstoff oder C₁-C₂₀-Alkyl steht,
R⁹ für Wasserstoff, C₁-C₂₀-Alkyl oder Aryl steht,
in allen ihren stereoisomeren und tautomeren Formen und Mischungen davon in allen Verhältnissen, und ihre physiologisch verträglichen Salze, Hydrate, und Ester.

2. Verbindungen der Formel (Ia) gemäß Anspruch 1, worin
R¹ für Wasserstoff, (C₁-C₁₀)-Alkyl, (C₃-C₈)-Cycloalkyl, Cycloalkylalkyl, Aryl oder (C₁-C₃)-Alkylaryl oder Arylalkyl steht, wobei die Alkylreste durch einen oder mehrere Substituenten R⁶ substituiert sein können,
R² unabhängig von R¹ für (C₁-C₁₀)-Alkyl, (C₃-C₈)-Cycloalkyl, Cycloalkylalkyl, Aryl oder (C₁-C₃)-Alkylaryl steht, wobei die Alkyl-reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann und der gegebenenfalls mit ein oder mehreren Resten R⁶ substituiert ist,
R³ für Wasserstoff, -CO-(C₁-C₇)-Alkyl, -CO-(C₁-C₃)-Alkylaryl oder -CO-Aryl steht,
R⁴ für (C₁-C₁₀)-Alkyl, Aryl oder (C₁-C₃)-Alkylaryl, -CO-O-(C₁-C₅)-Alkyl, -CO-O-Aryl, -CO-(C₁-C₅)-Alkyl oder -CO-Aryl steht, wobei die Alkylreste durch einen oder mehrere Substituenten R⁷ substituiert sein können,
R⁵ unabhängig von R³ eine der Bedeutungen von R³ hat,
R⁶ für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹,-COOH, -CO-O-(C₁-C₅)-Alkyl, -CO-O-Aryl, -S(O)ₙ-(C₁-C₅)-Alkyl, -SO₂-NR⁸R⁹ steht,
R⁷ unabhängig von R⁶ eine der Bedeutungen von R⁶ hat,
R⁸ für Wasserstoff oder (C₁-C₅)-Alkyl steht,
R⁹ für Wasserstoff, (C₁-C₅)-Alkyl oder Phenyl steht,
Aryl für Phenyl, Naphtyl oder Heteroaryl steht, die alle durch einen oder mehrere gleiche oder verschiedene Substituenten aus der Reihe Halogen, (C₁-C₅)-Alkyl, Phenyl, -OH, -O-(C₁-C₅)-Alkyl, (C₁-C₂)-Alkylendioxy, -NR⁸R⁹, -NO₂, -CO-(C₁-C₅)-Alkyl, -CF₃, -CN, -CONR⁸R⁹, -COOH, -CO-O-(C₁-C₅)-Alkyl, -S(O)ₙ-(C₁-C₅)-Alkyl, -SO₂-NR⁸R⁹ substituiert sein können,
Heteroaryl für einen 5- bis 7-gliedrigen ungesättigten Heterocyclus steht, der ein oder mehrerer Heteroatome aus der Reihe O, N, S enthält,
n für 0, 1 oder 2 steht,
in allen ihren stereoisomeren und tautomeren Formen und Mischungen davon in allen Verhältnissen und ihre physiologisch verträglichen Salze, Hydrate und Ester.

3. Verbindungen der Formel (Ia) gemäß Anspruch 1, worin
R¹ für Wasserstoff, (C₂-C₄)-Alkyl, das durch einen oder mehrere Substituenten R⁶ substituiert sein kann oder (C₁-C₂)-Alkylaryl,
R² für (C₂-C₄)-Alkyl, das durch einen oder mehrere Substituenten R⁶ substituiert sein kann, Cyclohexylmethyl oder (C₁-C₂)-Alkylaryl steht,
oder R¹ und R² zusammen mit dem sie tragenden Stickstoffatom einen 5-7-gliedrigen Ring bilden, der wahlweise kein oder ein weiteres Heteroatom aus der Reihe N, O, S enthält,
R³ für Wasserstoff, -CO-(C₁-C₃)-Alkyl oder -CO-Aryl,
R⁴ für Aryl, (C₁-C₅)-Alkyl oder -CO-O-Aryl, die jeweils durch einen oder mehrere Substituenten R⁷ substituiert sein können,
R⁵ für Wasserstoff,
R⁶ für -OH, -O-(C₁-C₃)-Alkyl, -NR⁸R⁹ oder -COOH,
R⁷ für -OH, (C₁-C₁₀)-Alkyloxy, Phenoxy oder Oxo,
Aryl für Phenyl, Thiophenyl, Furyl oder Pyridyl, die durch einen oder mehrere Substituenten aus der Reihe (C₁-C₃)-Alkyl, Halogen, (C₁-C₃)-Alkyloxy und (C₁-C₂)-Alkylendioxy substituiert sein können, steht, und
R⁸ und R⁹ die in Anspruch 1 angegebenen Bedeutungen haben,
in allen ihren stereoisomeren und tautomeren Formen und Mischungen davon in allen Verhältnissen und ihre physiologisch verträglichen Salze, Hydrate und Ester.

4. Verbindungen der Formel (Ia) gemäß Anspruch 1, worin
R¹ für Arylmethyl und
R² für Arylmethyl oder Cyclohexylmethyl steht
oder R¹ und R² zusammen mit dem sie tragenden Stickstoffatom einen Pyrrolidin-, Piperidin-, Morpholin-, Dimethylmorpholin-, Thiomorpholin, oder N-(C₁-C₂)-Alkylpiperazin-Ring bilden,
R³ für Wasserstoff,
R⁴ für Aryl oder 1,2-Dihydroxypropyl,
R⁵ für Wasserstoff
R⁶ für -OH, -O-(C₁-C₃)-Alkyl, -NR⁸R⁹ oder -COOH,
R⁷ für -OH, Decyloxy und Phenoxy,
Aryl für Phenyl, das durch einen oder mehrere Substituenten aus der Reihe (C₁-C₃)-Alkyl, Halogen und (C₁-C₃)-Alkyloxy und (C₁-C₂)-Alkylendioxy substituiert sein kann, steht, und
R⁸ und R⁹ die in Anspruch 1 angegebenen Bedeutungen haben,
in allen ihren stereoisomeren und tautomeren Formen und Mischungen davon in allen Verhältnissen und ihre physiologisch verträglichen Salze,Hydrate und Ester.

5. Verbindungen der Formel (Ia) gemäß Anspruch 1, die Tetrahydropteridine sind, worin R⁴ für Aryl, (C₁-C₅)-Alkyl oder -CO-O-Aryl, die jeweils durch einen oder mehrere Substituenten R⁷ substituiert sein können, steht.

6. Verbindungen der Formel (Ia) gemäß Anspruch 1, die Pteridine sind, worin R¹ und R² Alkyl und/oder Aryl sind oder worin R¹ Wasserstoff und R² Cycloalkyl oder Cycloalkylalkyl bedeuten, und worin R⁴ für Aryl, (C₁-C₅)-Alkyl oder -CO-O-Aryl, die jeweils durch einen oder mehrere Substituenten R⁷ substituiert sein können, steht.

7. Arzneimittel enthaltend eine Verbindung der Formel (Ia), (Ib) und/oder (Ic) gemäß Anspruch 1 neben den üblichen Hilfs- und Zusatzstoffen und gegebenenfalls weiteren Wirkstoffen.

8. Arzneimittel nach Anspruch 7 zur Therapie und Prophylaxe von Schlaganfällen, pathologischen Blutdruckabfällen, insbesondere beim septischen Schock und bei der Krebstherapie mit Cytokinen, Colitis Ulcerosa, Transplantatabstoßungsreaktionen, Nephritiden, Reperfusionsschäden, Infarkt-schäden, Kardiomyopathie, Morbus Alzheimer, Epilepsie, Migräne und Neuritiden unterschiedlicher Ätiogenese.

9. Arzneimittel nach Anspruch 7 zur Verwendung für diagnostische Zwecke.

10. Arzneimittel enthaltend eine
Verbindung der Formel I 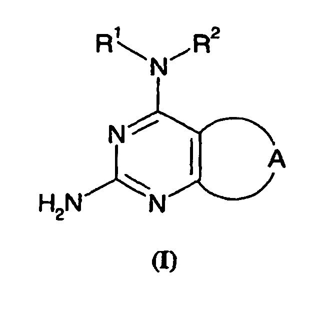 in der
A für eine Verbrückung der Form 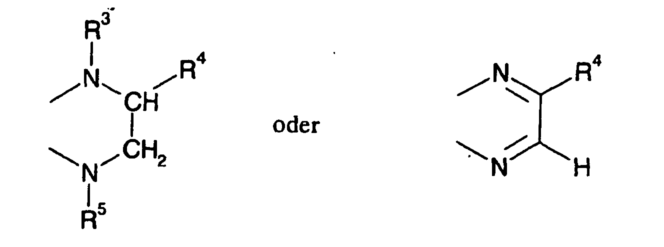 steht
R¹ für Wasserstoff, C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl, Alkylaryl oder Arylalkyl steht, wobei die organischen Reste durch einen oder mehrere Substituenten R⁶ substituiert sein können,
R² unabhängig von R¹ für C₁-C₂₀-Alkyl, C₂-C₂₀-Alkenyl, C₂-C₂₀₋Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl oder Arylalkyl steht, wobei die organischen Reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann, und der gegebenenfalls mit einem oder mehreren Resten R⁶ substituiert ist,
R³ für Wasserstoff, -CO-Alkyl, -CO-Alkylaryl oder -CO-Aryl steht,
R⁴ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl, Arylalkyl, -CO-O-Alkyl, -CO-O-Aryl, -CO-Alkyl, -CO-Aryl steht, wobei die organischen Reste durch einen oder mehrere Substituenten R⁷ substituiert sein können,
R⁵ unabhängig von R³ für Wasserstoff, -CO-Alkyl, -CO-Alkylaryl oder -CO-Aryl steht,
R⁶ für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹, -COOH, -CO-O-(C₁-C₅)-Alkyl, -CO-O-Aryl, -S(O)ₙ-(C₁-C₅)-Alkyl, -SO₂-NR⁸R⁹ steht,
R⁷ unabhängig von R⁶ eine der Bedeutungen von R⁶ hat,
R⁸ für Wasserstoff oder C₁-C₂₀-Alkyl steht,
R⁹ für Wasserstoff, C₁-C₂₀-Alkyl oder Aryl steht,
in allen ihren stereoisomeren und tautomeren Formen und Mischungen davon in allen Verhältnissen, und ihre physiologisch vertraglichen Salze, Hydrate, und Ester
als Hemmstoff der NO-Synthase.

11. Verfahren zur Herstellung einer Verbindung der Formel I 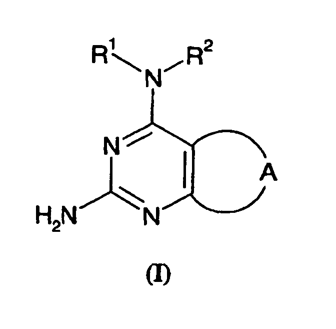 in der
A für eine Verbrückung der Form 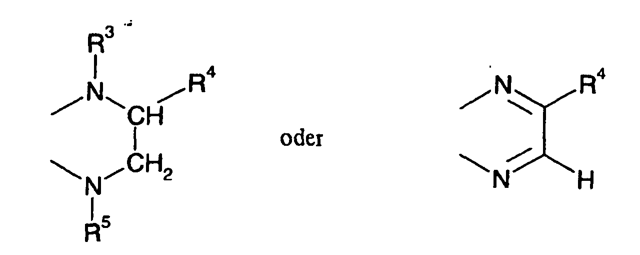 steht
R¹ für Wasserstoff, C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl, Alkylaryl oder Arylalkyl steht, wobei die organischen Reste durch einen oder mehrere Substituenten R⁶ substituiert sein können,
R² unabhängig von R¹ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl oder Arylalkyl steht, wobei die organischen Reste durch einen oder mehrere Substituenten R⁶ substituiert sein können,
R¹ und R², zusammen mit dem sie tragenden Stickstoffatom einen 3-8 gliedrigen Ring bilden können, der wahlweise 0, 1 oder 2 weitere Heteroatome aus der Reihe N, O, S enthalten kann, und der gegebenenfalls mit einem oder mehreren Resten R⁶ substituiert ist,
R³ für Wasserstoff, -CO-Alkyl, -CO-Alkylaryl oder -CO-Aryl steht,
R⁴ für C₁-C₂₀-Alkyl, C₂-C₂₀-Alkenyl, C₂-C₂₀-Alkinyl, Cycloalkyl mit drei bis acht Ringkohlenstoffatomen, Cycloalkenyl mit drei bis acht Ringkohlenstoffatomen, Cycloalkylalkyl mit fünf bis sechs Ringkohlenstoffatomen, Aryl oder Alkylaryl, Arylalkyl, -CO-O-Alkyl, -CO-O-Aryl, -CO-Alkyl, -CO-Aryl steht, wobei die organischen Reste durch einen oder mehrere Substituenten R⁷ substituiert sein können,
R⁵ unabhängig von R³ für Wasserstoff, -CO-Alkyl, -CO-Alkylaryl oder -CO-Aryl steht,
R⁶ für -F, -OH, -O-(C₁-C₁₀)-Alkyl, -O-Phenyl, -O-CO-(C₁-C₁₀)-Alkyl, -O-CO-Aryl, -NR⁸R⁹, Oxo, Phenyl, -CO-(C₁-C₅)-Alkyl, -CF₃, -CN, -CONR⁸R⁹,-COOH, -CO-O-(C₁-C₅)-Alkyl, -CO-O-Aryl, -S(O)ₙ-(C₁-C₅)-Alkyl, -SO₂-NR⁸R⁹ steht,
R⁷ unabhängig von R⁶ eine der Bedeutungen von R⁶ hat,
R⁸ für Wasserstoff oder C₁-C₂₀-Alkyl steht,
R⁹ für Wasserstoff, C₁-C₂₀-Alkyl oder Aryl steht,
in allen ihren stereoisomeren und tautomeren Formen und Mischungen davon in allen Verhältnissen, und ihre physiologisch verträglichen Salze, Hydrate, und Ester,
indem man
eine Verbindung der Formel II 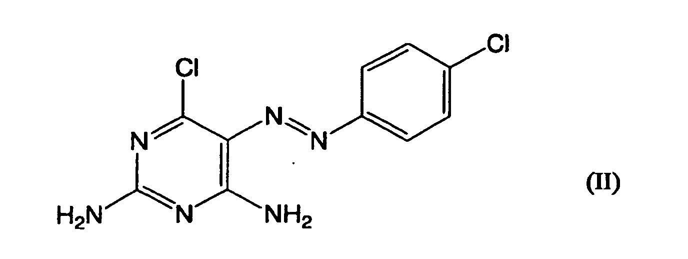 mit einer Verbindung der Formel III
HNR¹R² (III)
zu einer Verbindung der Formel IV 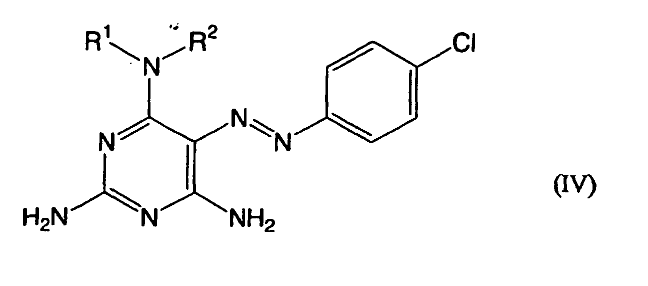 umsetzt und diese durch katalytische Hydrierung in eine Verbindung der Formel V 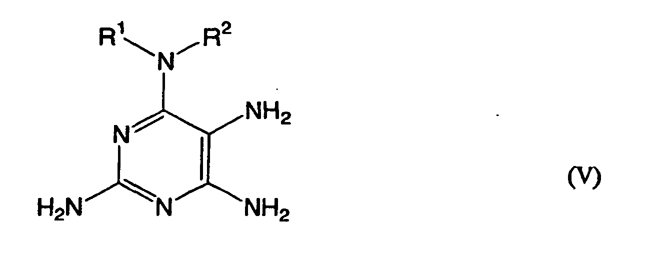 überführt, welche mit einer Verbindung der Formel VI 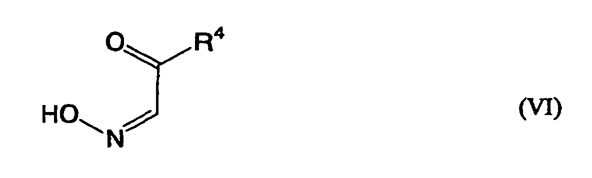 zu einer Verbindung der Formel I umgesetzt wird, welche durch geeignete Derivatisierung, bevorzugt Acylierung, in die gewünschte Verbindung der Formel I oder deren physiologisch verträglichen Salze, Hydrate, Ester und Addukte überführt werden kann, und worin die Substituenten die in den Ansprüche 1 bis 3 genannten Bedeutungen haben.

## Claims

1. Compounds of the formulae (Ia), (Ib) and (Ic), 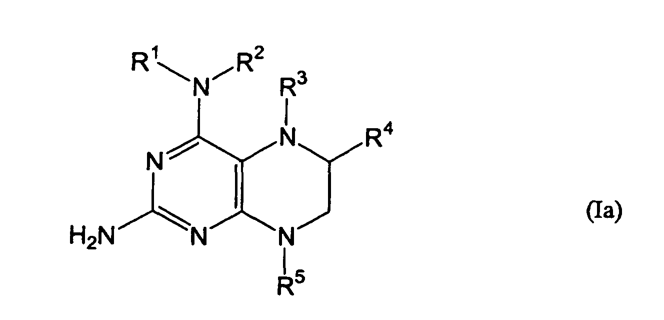 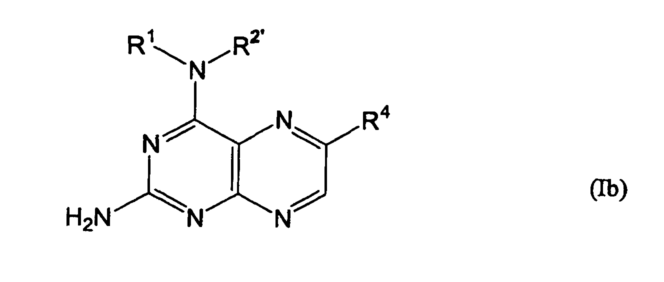 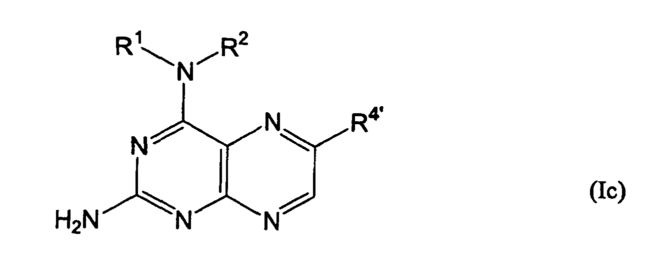 in which
R¹ is hydrogen, C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, aryl, alkylaryl, arylalkyl, wherein the organic radicals can be substituted with one or more substituents R⁶,
R² is independently from R¹ C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, alkylaryl, or arylalkyl, wherein the organic radicals can be substituted with one or more substituents R⁶,
R^{2'} is independently from R¹ C₂-C₂₀-alkenyl, C₂-C₂₀-alkynyl, cycloalkenyl with three to eight ring carbon atoms, cycloalkylalkyl with five to six ring carbon atoms, or heteroaryl, wherein the organic radicals can be substituted with one or more substituents R⁶,
R¹ and R², together with the nitrogen atom bearing them, may form a 3-8-membered ring which may optionally comprise 0, 1 or 2 further heteroatoms chosen from N, O, and S, wherein said ring is optionally substituted with one or more substituents R⁶,
R³ is hydrogen, -CO-alkyl, -CO-alkylaryl or -CO-aryl,
R⁴ is C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, or alkylaryl, arylalkyl, -CO-O-alkyl, -CO-O-aryl, -CO-alkyl, -CO-O-aryl, wherein the organic radicals can be substituted with one or more substituents chosen from R⁷,
R^{4'} is C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, heteroaryl, or -CO-O-alkyl, -CO-O-aryl, -CO-alkyl, -CO-aryl, wherein the organic radicals can be substituted with one or more substituents R⁷,
R⁵ is independently from R³ hydrogen, -CO-alkyl, -CO-alkylaryl, or -CO-aryl,
R⁶ is independently from R⁶ -F, -OH, -O-(C₁-C₁₀)-alkyl, -O-phenyl, -O-CO-(C₁-C₁₀)-alkyl, -O-CO-aryl, -NR⁸R⁹, oxo, phenyl, -CO-(C₁-C₅)-alkyl, -CF₃, -CN,-CONR⁸R⁹, -COOH, -CO-O-(C₁-C₅)-alkyl, -CO-O-aryl, -S(O)ₙ-(C₁-C₅)-alkyl,-SO₂-NR⁸R⁹,
R⁷ has independently from R⁶ one of the meanings of R⁶,
R⁸ is hydrogen or C₁-C₂₀-alkyl, and
R⁹ is hydrogen, C₁-C₂₀-alkyl or aryl,
in any stereoisomeric or tautomeric forms or mixtures thereof in all ratios and their physiologically acceptable salts, hydrates and esters.

2. Compounds of the formula (Ia) as claimed in claim 1, in which
R¹ is hydrogen, (C₁-C₁₀)-alkyl, (C₃-C₈)-cycloalkyl, cycloalkylalkyl, aryl, or (C₁-C₃)-alkylaryl or arylalkyl, wherein the alkyl radicals can be substituted with one or more substituents R⁶,
R² is independently from R¹ (C₁-C₁₀)-alkyl, (C₃-C₈)-cycloalkyl, cycloalkylalkyl, aryl, or (C₁-C₃)-alkylaryl, wherein the alkyl radicals can be substituted with one or more substituents R⁶,
R¹ and R² may, together with the nitrogen atom bearing them, form a 3-8-membered ring, wherein said 3-8-membered ring may optionally comprise 0, 1 or 2 further heteroatoms chosen from N, O, and S and wherein said 3-8-membered ring is optionally substituted with one or more substituents R⁶,
R³ is hydrogen, -CO-(C₁-C₇)-alkyl, -CO-(C₁-C₃)-alkylaryl, or -CO-aryl,
R⁴ is (C₁-C₁₀)-alkyl, aryl or (C₁-C₃)-alkylaryl, -CO-O-(C₁-C₅)-alkyl, -CO-O-aryl, -CO-(C₁-C₅)-alkyl or -CO-aryl, wherein the alkyl radicals can be substituted with one or more substituents R⁷,
R⁵ has independently from R⁶ one of the meanings of R³,
R⁶ is -F, -OH, -O-(C₁-C₁₀)-alkyl, -O-phenyl, -O-CO-(C₁-C₁₀)-alkyl, -O-CO-aryl, -NR⁸R⁹, oxo, phenyl, -CO-(C₁-C₅)-alkyl, -CF₃, -CN, -CONR⁸R⁹, -COOH, -CO-O-(C₁-C₅)-alkyl, -CO-O-aryl, -S(O)ₙ-(C₁-C₅)-alkyl, -SO₂-NR⁸R⁹,
R⁷ has independently from R⁶ one of the meanings of R⁶,
R⁸ is hydrogen or (C₁-C₅)-alkyl, and
R⁹ is hydrogen, (C₁-C₅)-alkyl or phenyl,
Aryl is phenyl, naphthyl or heteroaryl, which can all be substituted by one or more substituents which can be same or different and chosen from halogen, (C₁-C₅)-alkyl, phenyl, -OH, -O-(C₁-C₅)-alkyl, (C₁-C₂)-alkylenedioxy, -N⁸R⁹, -NO₂, -CO-(C₁-C₅)-alkyl, -CF₃, -CN, -CONR⁸R⁹, -COOH, -CO-O-(C₁-C₅)-alkyl, -S(O)n-(C₁-C₅)-alkyl, -SO₂-NR⁸R⁹,
heteroaryl is a 5-7-membered ring unsaturated heterocycle which contains one or more heteroatoms chosen from N, O, and S,
n is 0, 1 or 2,
in any stereoisomeric or tautomeric forms or mixtures thereof in all ratios and their physiologically acceptable salts, hydrates and esters.

3. Compounds of the formula (Ia) as claimed in claim 1, in which
R¹ is hydrogen, (C₂-C₄)-alkyl which can be substituted by one or more substiutents R⁶, or (C₁-C₂)-alkylaryl,
R² is (C₂-C₄)-alkyl which can be substituted by one or more substituents R⁶, cyclohexylmethyl or (C₁-C₂)-alkylaryl,
or R¹ and R² may, together with the nitrogen atom bearing them, form a 5-7-membered ring wherein said 5-7-membered ring optionally comprises none or a further heteroatom chosen from N, O, and S,
R³ is hydrogen, -CO-(C₁-C₃)-alkyl, or -CO-aryl,
R⁴ is aryl, heteroaryl, (C₁-C₅)-alkyl, -CO-O-aryl or -CO-heteroaryl, which all can optionally be substituted with one or more substituents R⁷,
R⁵ is hydrogen,
R⁶ is -OH, -O-(C₁-C₃)-alkyl, -NR⁸R⁹ or -COOH,
R⁷ is -OH, (C₁-C₁₀)-alkyloxy, phenoxy or oxo,
Aryl is phenyl, thiophenyl, furyl and pyridyl, which can be substituted by one or more substituents chosen from (C₁-C₃)-alkyl, halogen, (C₁-C₃)-alkyloxy and (C₁-C₂)-alkylenedioxy, and
R⁸ and R⁹ have the meanings as given in claim 1
in any stereoisomeric or tautomeric forms or mixtures thereof in all ratios and their physiologically acceptable salts, hydrates and esters.

4. Compounds of the formula (Ia) as claimed in claim 1, wherein
R¹ is arylmethyl,
R² is arylmethyl or cyclohexylmethyl,
or R¹ and R², together with the nitrogen atom bearing them, form a pyrrolidine, piperidine, morpholine, dimethylmorpholine, thiomorpholine, or N-(C₁-C₂)-alkylpiperazine ring,
R³ is hydrogen,
R⁴ is aryl or 1,2-dihydroxypropyl,
R⁵ is hydrogen,
R⁶ is -OH, -O-(C₁-C₃)-alkyl, -NR⁸R⁹ or -COOH, and
R⁷ is -OH, decyloxy or phenoxy,
Aryl is phenyl, which can be substituted by one or more substituents chosen from (C₁-C₃)-alkyl, halogen and (C₁-C₃)-alkyloxy and (C₁-C₂)-alkylenedioxy,
R⁸ and R⁹ have the meanings as given in claim 1
in any stereoisomeric or tautomeric forms or mixtures thereof in all ratios and their physiologically acceptable salts, hydrates and esters.

5. Compounds of formula (Ia) as claimed in claim 1, which are tetrahydropteridines, wherein R⁴ is aryl, (C₁-C₅)-alkyl or -CO-O-aryl, each of which can be substituted with one or more substituents R⁷.

6. Compounds of formula (Ia) as claimed in claim 1, which are pteridines, wherein R¹ and R² are each alkyl or aryl, or wherein R¹ is hydrogen and R² is cycloalkyl or cycloalkylalkyl, and wherein R⁴ is aryl, (C₁-C₅)-alkyl or -CO-O-aryl, each of which can be substituted with one or more substituents R⁷.

7. A pharmaceutical comprising a compound of formula (Ia), (Ib) or (Ic) as claimed in claim 1 and an additional ingredient chosen from conventional excipients and additives and optionally further drugs.

8. Pharmaceutical according to claim 7 for therapy and prevention of strokes, pathological falls in blood pressure, in particular in septic shock or cancer therapy with cytokines, ulcerative colitis, transplant rejection reactions, nephritis, reperfusion damage, infarct damage, cardiomyopathy, Alzheimer's disease, epilepsy, migraine and neuritis of varying etiogenesis.

9. Pharmaceutical according to claim 7 for use in diagnostic purposes.

10. A pharmaceutical comprising a compound of formula I 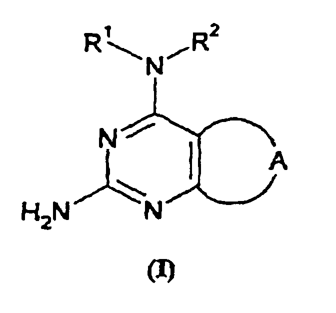 in which A is a bridge of the form 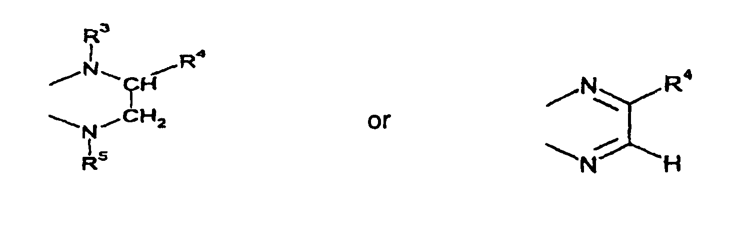
R¹ is hydrogen, C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, alkylaryl or arylalkyl, wherein the organic radicals can be substituted with one or more substituents R⁶,
R² is independently from R¹ C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, alkylaryl, or arylalkyl, wherein the organic radicals can be substituted with at one or more substituents R⁶,
R¹ and R², together with the nitrogen atom bearing them, may form a 3-8-membered ring which may optionally comprise 0, 1 or 2 further heteroatoms chosen from N, O, and S, wherein said ring is optionally substituted with one or more substituents R⁶,
R³ is hydrogen, -CO-alkyl, -CO-alkylaryl or -CO-aryl,
R⁴ is C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, or alkylaryl, arylalkyl, -CO-O-alkyl, -CO-O-aryl, -CO-alkyl, -CO-O-aryl, wherein the organic radicals can be substituted with one or more substituents R⁷,
R⁵ is independently from R³ hydrogen, -CO-alkyl, -CO-alkylaryl, or -CO-aryl,
R⁶ is independently from R⁶ -F, -OH, -O-(C₁-C₁₀)-alkyl, -O-phenyl, -O-CO-(C₁-C₁₀)-alkyl, -O-CO-aryl, -NR⁸R⁹, oxo, phenyl, -CO-(C₁-C₅)-alkyl, -CF₃, -CN, -CONR⁸R⁹, -COOH, -CO-O-(C₁-C₅)-alkyl, -CO-O-aryl, -S(O)ₙ-(C₁-C₅)-alkyl, -SO₂-NR⁸R⁹,
R⁷ has independently from R⁶ one of the meanings of R⁶,
R⁸ is hydrogen or C₁-C₂₀-alkyl, and
R⁹ is hydrogen, C₁-C₂₀-alkyl or aryl,
in any stereoisomeric or tautomeric forms or mixtures thereof in all ratios and their physiologically acceptable salts, hydrates and esters
as inhibitors of NO synthase.

11. A process for preparing a compound of formula I 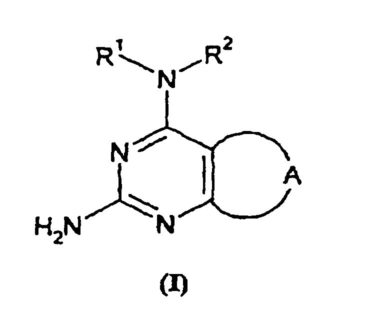 in which
A is a bridge of the form 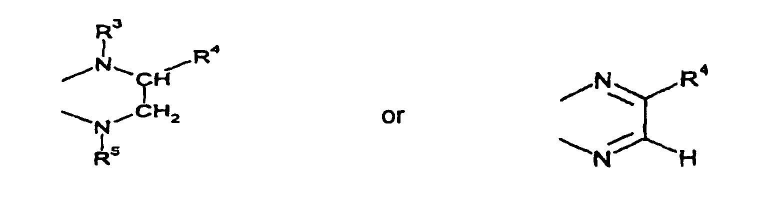
R¹ is hydrogen, C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, alkylaryl, arylalkyl, wherein the organic radicals can be substituted with one or more substituents R⁶,
R² is independently from R¹ C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, alkylaryl, or arylalkyl, wherein the organic radicals can be substituted with at one or more substituents R⁶,
R¹ and R², together with the nitrogen atom bearing them, may form a 3-8-membered ring which may optionally comprise 0, 1 or 2 further heteroatoms chosen from N, O, and S, wherein said ring is optionally substituted with one or more substituents R⁶,
R³ is hydrogen, -CO-alkyl, -CO-alkylaryl or -CO-aryl,
R⁴ is C₁-C₂₀-alkyl, C₂-C₂₀-alkenyl, C₂-C₂₀-alkynyl, cycloalkyl with three to eight ring carbon atoms, cycloalkenyl with three to eight ring carbon atoms, cycloalkylalkyl with five to six ring carbon atoms, aryl, or alkylaryl, arylalkyl, -CO-O-alkyl, -CO-O-aryl, -CO-alkyl, -CO-O-aryl, wherein the organic radicals can be substituted with one or more substituents R⁷,
R⁵ is independently from R³ hydrogen, -CO-alkyl, -CO-alkylaryl, or -CO-aryl,
R⁶ is independently from R⁶-F, -OH, -O-(C₁-C₁₀)-alkyl, -O-phenyl, -O-CO-(C₁-C₁₀)-alkyl, -O-CO-aryl, -NR⁸R⁹, oxo, phenyl, -CO-(C₁-C₅)-alkyl, -CF₃, -CN, -CONR⁸R⁹, -COOH, -CO-O-(C₁-C₅)-alkyl, -CO-O-aryl, -S(O)ₙ-(C₁-C₅)-alkyl, -SO₂-NR⁸R⁹,
R⁷ has independently from R⁶ one of the meanings of R⁶,
R⁸ is hydrogen or C₁-C₂₀-alkyl, and
R⁹ is hydrogen, C₁-C₂₀-alkyl or aryl,
in any stereoisomeric or tautomeric forms or mixtures thereof in all ratios and their physiologically acceptable salts, hydrates and esters
which process comprising reacting a compound of the formula II 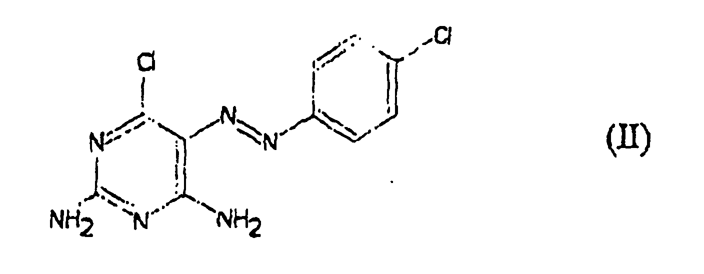 with a compound of the formula III
HNR¹R² (III)
which results in a compound of the formula IV 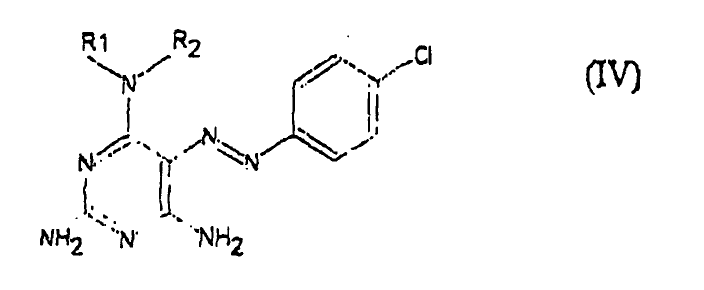 wherein the compound of formula IV is converted to a compound of formula V by catalytic hydrogenation 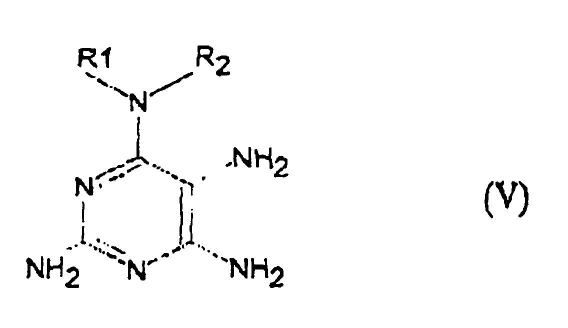 and wherein the compound of formula V is reacted with a compound of the formula VI 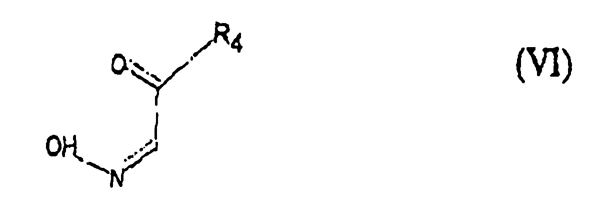 to give a compound of formula I, which can be converted by suitable derivatisation, preferably acylation, into the desired compound of formula I or physiologically acceptable salts, hydrates, esters and adducts, and wherein the substituents have the meanings as given in any of claims 1 to 3.

## Revendications

1. Composés des formules (Ia), (Ib) et (Ic) : 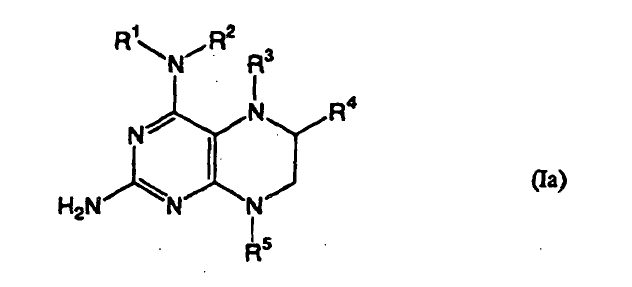 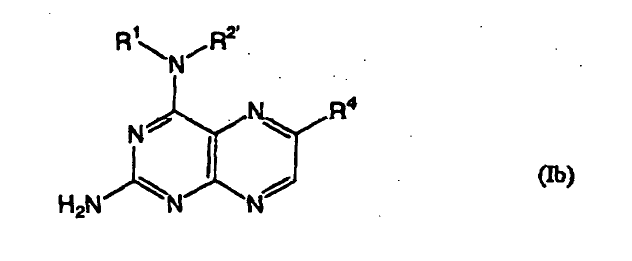 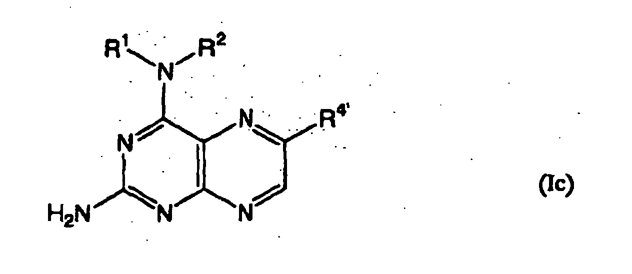 où
R¹ représente un hydrogène, alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, les groupes organiques pouvant être substitués par un ou plusieurs substituants R⁶,
R² représente, indépendamment de R¹, un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, ou arylalkyle, les groupes organiques pouvant être substitués par un ou plusieurs substituants R⁶,
R^{2'} représente, indépendamment de R¹, alcényle en C₂-C₂₀, alcynyle en C₂-C₂₀, cycloalcényle avec 3 à 8 atomes de carbone cycliques, cycloalkylalkyle avec 5 à 6 atomes de carbone cycliques, ou hétéroaryle, les groupes organiques pouvant être substitués par un ou plusieurs substituants R⁶,
R¹ et R² peuvent former, avec l'atome d'azote qui les porte, un cycle ayant 3 à 8 chaînons, qui peut contenir au choix, 0, 1 ou 2 autres hétéroatomes de la série N, O, S, et qui est éventuellement substitué par un ou plusieurs substituts R⁶,
R³ représente un hydrogène, -CO-alkyle, -CO-alkylaryle ou -CO-aryle,
R⁴ représente un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, -CO-O-alkyle, -CO-O-aryle, -CO-alkyle ou -CO-aryle, les groupes organiques pouvant être substitués par un ou plusieurs substituants R⁷,
R^{4'} représente un alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone hétéroaryle ou -CO-O-alkyle, -CO-O-aryle, -CO-alkyle, -CO-aryle, les restes organiques pouvant être substitués par un ou plusieurs substituants R⁷,
R⁵ représente, indépendamment de R³, un hydrogène, -CO-alkyle, -CO-alkylaryle ou -CO-aryle,
R⁶ représente F, OH, O-alkyle (C₁-C₁₀), O-phényle, O-CO-alkyle (C₁-C₁₀), O-CO-aryle, NR⁸R⁹, oxo, phényle, CO-alkyle (C₁-C₅), CF₃, CN, CONR⁸R⁹, COOH, CO-O-alkyle (C₁-C₅), CO-O-aryle, S(O)ₙ-alkyle (C₁-C₅), SO₂-NR⁸R⁹,
R⁷ a, indépendamment de R⁶, une des significations de R⁶,
R⁸ représente un hydrogène ou alkyle en C₁-C₂₀,
R⁹ représente un hydrogène, alkyle en C₁-C₂₀ ou aryle,
sous toutes leurs formes stéréoisomères et tautomères, ainsi que leurs mélanges en tout rapport, et leurs sels, hydrates et esters physiologiquement compatibles.

2. Composés selon la formule (la) selon la revendication 1, où
R¹ représente un hydrogène, alkyle en C₁-C₁₀, cycloalkyle en C₃-C₈, cycloalkylalkyle, aryle ou alkyl(C₁-C₃)aryle ou arylalkyle, les groupes alkyle pouvant être substitués par un ou plusieurs substituants R⁶,
R² représente, indépendamment de R¹, un alkyle en C₁-C₁₀, cycloalkyle en C₃-C₈, cycloalkylalkyle, aryle ou alkyl(C₁-C₃)aryle ou arylalkyle, les groupes alkyle pouvant être substitués par un ou plusieurs substituants R⁶,
R¹ et R² peuvent former, avec l'atome d'azote qui les porte, un cycle ayant 3 à 8 chaînons, qui peut contenir au choix, 0, 1 ou 2 autres hétéroatomes de la série N, O, S et qui est éventuellement substitué par un ou plusieurs restes R⁶,
R³ représente un hydrogène, -CO-alkyle(C₁-C₇), -CO-alkyl(C₁-C₃)aryle ou-CO-aryle,
R⁴ représente un alkyle en C₁-C₁₀, aryle ou alkyl(C₁-C₃)aryle, -CO-O-alkyle (C₁-C₅), -CO-O-aryle, -CO-alkyle (C₁-C₅) ou -CO-aryle, où les restes alkyle peuvent être substitués par un ou plusieurs substituants R⁷,
R⁵ a, indépendamment de R³, une des significations de R³,
R⁶ représente F, OH, O-alkyle (C₁-C₁₀), O-phényle, O-CO-alkyle (C₁-C₁₀), O-CO-aryle, NR⁸R⁹, oxo, phényle, CO-alkyle (C₁-C₅), CF₃, CN, CONR⁸R⁹, COOH, CO-O-alkyle (C₁-C₅), CO-O-aryle, S(O)ₙ-alkyle (C₁-C₅), SO₂-NR⁸R⁹,
R⁷ a, indépendamment de R⁶, une des significations de R⁶,
R⁸ représente un hydrogène ou alkyle en C₁-C₅,
R⁹ représente un hydrogène, alkyle en C₁-C₅ ou phényle, un aryle désignant un phényle, naphtyle ou hétéroaryle, qui peuvent tous trois être substitués par un ou plusieurs substituants, identiques ou différents, de la série des halogènes, alkyle (C₁-C₅), phényle, OH, O-alkyle (C₁-C₅), alkylènedioxy (C₁-C₂), NR⁸R⁹, NO₂, CO-alkyle (C₁-C₅), CF₃, CN, CONR⁸R⁹, COOH, CO-O-alkyle (C₁-C₅), S(O)ₙ-alkyle (C₁-C₅), SO₂-NR⁸R⁹,
un hétéroaryle désignant hétérocycle insaturé de 5 à 7 membres, qui contient un ou plusieurs hétéroatomes de la série O, N, S,
n représente 0, 1 ou 2,
sous toutes leurs formes stéréoisomères et tautomères, ainsi que leurs mélanges en tout rapport, et leurs sels, hydrates et esters physiologiquement compatibles.

3. Composés selon la formule (Ia) selon la revendication 1, où
R¹ représente un hydrogène, un alkyle en C₂-C₄, qui peut être substitué par un ou plusieurs substituants R⁶, ou un alkyl(C₁-C₂)aryle,
R² représente un alkyle en C₂-C₄, qui peut être substitué par un ou plusieurs substituants R⁶, un cyclohexylméthyle ou un alkyl(C₁-C₂)aryle,
ou bien R¹ et R² peuvent former, avec l'atome d'azote qui les porte, un cycle ayant 5 à 7 chaînons, qui peut contenir aucun ou un autre hétéroatome de la série N, O, S,
R³ représente un hydrogène, -CO-alkyle(C₁-C₃), ou -CO-aryle,
R⁴ représente un aryle, alkyle en C₁-C₅, ou -CO-O-aryle, qui peuvent être substitués par un ou plusieurs substituants R⁷,
R⁵ représente un hydrogène,
R⁶ représente un OH, O-alkyle en C₁-C₃, NR⁸R⁹, ou COOH,
R⁷ représente un OH, alkyloxy en C₁-C₁₀, phénoxy ou oxo,
un aryle représentant un phényle, thiophényle, furyle ou pyridyle, qui peuvent être substitués par un ou plusieurs substituants, identiques ou différents, de la série alkyle en C₁-C₃, halogène, alkyloxy en C₁-C₃ et alkylènedioxy en C₁-C₂, et
R⁸ et R⁹ ont les significations données dans la revendication 1,
sous toutes leurs formes stéréoisomères et tautomères, ainsi que leurs mélanges en tout rapport, et leurs sels, hydrates et esters physiologiquement compatibles.

4. Composés selon la formule (Ia) selon la revendication 1, où
R¹ représente un arylméthyle,
R² représente un arylméthyle ou un cyclohexylméthyle,
ou bien R¹ et R² forment, avec l'atome d'azote qui les porte, un cycle pyrrolidine, pipéridine, morpholine, diméthylmorpholine, thiomorpholine ou N-alkyl(C₁-C₂)pipérazine,
R³ représente hydrogène,
R⁴ représente un aryle ou 1,2-dihydroxypropyle,
R⁵ représente un hydrogène,
R⁶ représente un OH, O-alkyle (C₁-C₃), NR⁸R⁹, ou COOH,
R⁷ représente un OH, décyloxy et phénoxy,
un aryle représentant un phényle, qui peut être substitué par un ou plusieurs substituants, identiques ou différents, de la série alkyle (C₁-C₃), halogène, alkyloxy (C₁-C₃) et alkylènedioxy (C₁-C₂), et
R⁸ et R⁹ ont les significations données à la revendication 1,
sous toutes leurs formes stéréoisomères et tautomères, ainsi que leurs mélanges en tout rapport, et leurs sels, hydrates et esters physiologiquement compatibles.

5. Composés de la formule (Ia) selon la revendication 1, qui sont des tétrahydroptéridines, où R⁴ représente un aryle, un alkyle (C₁-C₅) ou CO-O-aryle, qui peuvent chaque fois, être substitués par un ou plusieurs substituants R⁷.

6. Composés de la formule (Ia) selon la revendication 1, qui sont des ptéridines, où R¹ et R² sont alkyle et/ou aryle ou où R¹ est un hydrogène et R² est un cycloalkyle ou un cycloalkylaklyle, et où R⁴ représente un aryle, alkyle (C₁-C₅) ou CO-O-aryle, qui peuvent chaque fois, être substitués par un ou plusieurs substituants R⁷.

7. Médicament contenant un composé de la formule (Ia), (Ib) et/ou (Ic) selon la revendication 1, en plus d'adjuvants et additifs usuels et éventuellement échéant, d'autres agents actifs.

8. Médicament selon la revendication 7, pour la thérapie et la prophylaxie d'attaques cardiaques, de chutes pathologiques de la tension artérielle, en particulier lors de chocs septiques et lors de thérapies cancéreuses avec des cytokines, de colites ulcéreuses, de réactions de rejet de transplantations, de néphrites, de lésions de la reperfusion, de lésions d'infarctus, de la cardiomyopathie, de la maladie d'Alzheimer, de l'épilepsie, de migraines et de névrites de différentes étiologies.

9. Médicament selon la revendication 7, à utiliser pour des objets diagnostiques.

10. Médicament contenant un composé de la formule 1 : 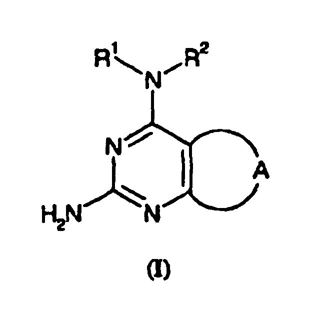 dans laquelle
A représente un pontage 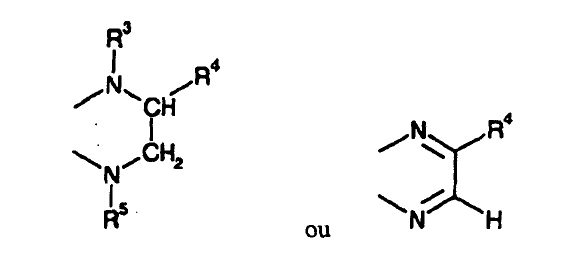
R¹ représente un hydrogène, alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, les groupes organiques pouvant être substitués par un ou plusieurs substituants R⁶,
R² représente, indépendamment de R¹, un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, ou arylalkyle, les groupes organiques pouvant être substitués par un ou plusieurs substituants R⁶,
R¹ et R² peuvent former, avec l'atome d'azote qui les porte, un cycle ayant de 3 à 8 chaînons, qui peut contenir au choix, 0, 1 ou 2 autres hétéroatomes de la série N, O, S et qui est éventuellement substitué par un ou plusieurs groupes R⁶,
R³ représente un hydrogène, -CO-alkyle, -CO-alkylaryle ou -CO-aryle,
R⁴ représente un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, -CO-O-alkyle, -CO-O-aryle, -CO-alkyle ou -CO-aryle, les groupes organiques pouvant être substitués par un ou plusieurs substituants R⁷,
R⁵ représente, indépendamment de R³, hydrogène, -CO-alkyle, -CO-alkylaryle ou -CO-aryle,
R⁶ représente F, OH, O-alkyle (C₁-C₁₀), O-phényle, O-CO-alkyle (C₁-C₁₀), O-CO-aryle, NR⁸R⁹, oxo, phényle, CO-alkyle (C₁-C₅), CF₃, CN, CONR⁸R⁹, COOH, CO-O-alkyle (C₁-C₅), CO-O-aryle, S(O)ₙ-alkyle (C₁-C₅), SO₂-NR⁸R⁹,
R⁷ a, indépendamment de R⁶, une des significations de R⁶,
R⁸ représente un hydrogène ou alkyle en C₁-C₂₀,
R⁹ représente un hydrogène, alkyle en C₁-C₂₀ ou aryle,
sous toute forme stéréoisomère et tautomère, ainsi que des mélanges en tout rapport, et des sels, hydrates et esters physiologiquement compatibles, à titre d'inhibiteur de la NO synthétase.

11. Procédé de préparation d'un composé de la formule I, 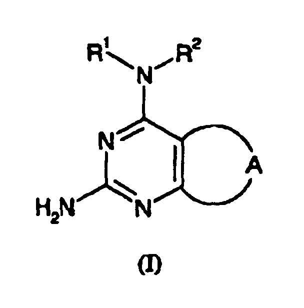 dans laquelle
A représente un pontage 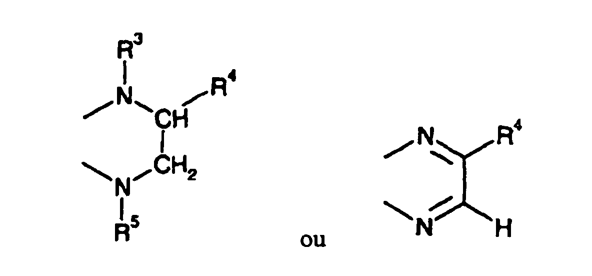
R¹ représente un hydrogène, alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, les groupes organiques pouvant être substitués par un ou plusieurs substituants R⁶,
R² représente, indépendamment de R¹, un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, ou arylalkyle, les groupes organiques pouvant être substitués par un ou plusieurs substituants R⁶,
R¹ et R² peuvent former , avec l'atome d'azote qui les porte, un cycle ayant de 3 à 8 chaînons, qui peut contenir au choix, 0, 1 ou 2 autres hétéroatomes de la série N, O, S et qui est éventuellement, est substitué par un ou plusieurs restes R⁶,
R³ représente un hydrogène, -CO-alkyle, -CO-alkylaryle ou -CO-aryle,
R⁴ représente un alkyle en C₁-C₂₀, alcényle en C₂-C₂₀, alcynyle en C₂-C₂₀, cycloalkyle avec 3 à 8 atomes de carbone cycliques, cycloalcényle avec 3 à 8 atomes de carbone cycliques, cycloalkylalkyle avec 5 à 6 atomes de carbone cycliques, aryle, alkylaryle, arylalkyle, -CO-O-alkyle, -CO-O-aryle, -CO-alkyle ou -CO-aryle, les groupes organiques pouvant être substitués par un ou plusieurs substituants R⁷,
R⁵ représente, indépendamment de R³, un hydrogène, -CO-alkyle, -CO-alkylaryle ou -CO-aryle,
R⁶ représente F, OH, O-alkyle (C₁-C₁₀), O-phényle, O-CO-alkyle (C₁-C₁₀), O-CO-aryle, NR⁸R⁹, oxo, phényle, CO-alkyle (C₁-C₅), CF₃, CN, CONR⁸R⁹, COOH, CO-O-alkyle (C₁-C₅), CO-O-aryle, S(O)ₙ-alkyle (C₁-C₅), SO₂-NR⁸R⁹,
R⁷ a, indépendamment de R⁶, une des significations de R⁶,
R⁸ représente un hydrogène ou alkyle en C₁-C₂₀,
R⁹ représente un hydrogène, alkyle en C₁-C₂₀ ou aryle,
sous toute forme stéréoisomère et tautomère, ainsi que des mélanges en tout rapport, et des sels, hydrates et esters physiologiquement compatibles,
dans lequel on fait réagir un composé de formule II : 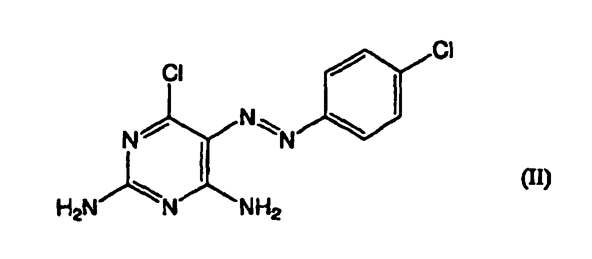 avec un composé de formule III :
HNR¹R² (III)
pour former un composé de formule IV : 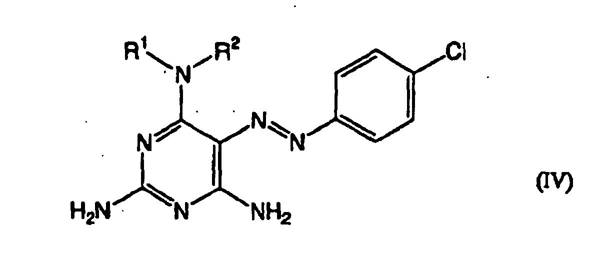 et où ce composé est converti, par hydrogénation catalytique, en un composé de formule V : 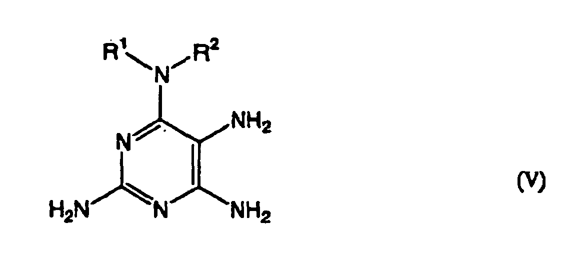 qui est mis à réagir avec un composé de formule VI : 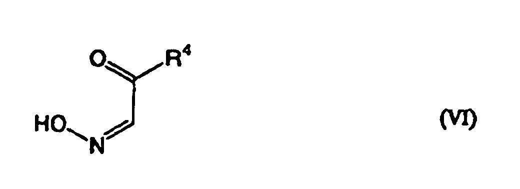 pour former un composé de formule I, qui peut être converti par une substitution appropriée, de préférence une acylation, en le composé souhaité de la formule I, ou un de ses sels, hydrates, esters et adduits physiologiquement compatibles, et où les substituants ont les significations données dans les revendications 1 à 3.
